# EUROPÄISCHE PATENTSCHRIFT

(11) **EP 1 740 555 B1**
(45) Veröffentlichungstag und Bekanntmachung des Hinweises auf die Patenterteilung: **13.06.2012**
(21) Anmeldenummer: 05716559.9
(22) Anmeldetag: 08.04.2005
(51) Int. Cl.: C07D 251/70, C08F 2/00

(54) **VINYL-, METHACRYLOYL- ODER ACRYLOLYL-GRUPPEN ENTHALTENDE STRAHLUNGSHÄRTBARE 1,3,5-TRIAZINCARBAMATE UND -HARNSTOFFE**
RADIATION CURABLE 1,3,5-TRIAZINE CARBAMATES AND 1,3,5-TRIAZINE UREAS CONTAINING VINYL GROUPS, METHACRYLOYL GROUPS, OR ACRYLOYL GROUPS
1,3,5-TRIAZINECARBAMATES ET 1,3,5-TRIAZINE-UREES DURCISSABLES PAR RAYONNEMENT, RENFERMANT DES GROUPES VINYLE, METHACRYLOYLE OU ACRYLOLYLE

(30) Priorität: 14.04.2004 DE 102004018546
(43) Veröffentlichungstag der Anmeldung: 10.01.2007
(73) Patentinhaber: BASF SE, 67056 Ludwigshafen (DE)
(72) Erfinder: HEISCHKEL, Yvonne, 68199 Mannheim (DE); SCHNEIDER, Jörg, 69469 Weinheim (DE); SCHWALM, Reinhold, 67157 Wachenheim (DE); SCHERR, Günter, 67065 Ludwigshafen (DE)
(86) Internationale Anmeldenummer: PCT/EP2005/003687
(87) Internationale Veröffentlichungsnummer: WO 2005/103016

(56) Entgegenhaltungen:
- DATABASE CAPLUS CHEMICAL ABSTRACTS SERVICE, COLUMBUS, OHIO, US; 1975, XP002340678 Database accession no. 1975:44126 & KEHR, CLIFTON L. ET AL: "Thiol/ene curable polymer technology" PAPERS PRESENTED AT [THE] MEETING - AMERICAN CHEMICAL SOCIETY, DIVISION OF ORGANIC COATINGS AND PLASTICS CHEMISTRY , 33(1), 295-302 CODEN: ACOCAO; ISSN: 0096-512X, 1973,
- PATENT ABSTRACTS OF JAPAN Bd. 2003, Nr. 12, 5. Dezember 2003 (2003-12-05) -& JP 2003 280187 A (FUJI PHOTO FILM CO LTD), 2. Oktober 2003 (2003-10-02)

## Beschreibung

Die vorliegende Erfindung beschreibt strahlungshärtbare 1,3,5-Triazincarbamate und -harnstoffe, Verfahren zu deren Herstellung und deren Verwendung.

Die Darstellung von 1,3,5-Triazincarbamaten ist in DE-A1 101 51 564 oder WO 97/08235, S. 3, Z. 9 - 22 beschrieben. Die dort genannten Darstellungswege führen zu alkylsubstituierten 1,3,5-Triazincarbamaten. Diese Methoden sind aufgrund des stark basischen Reaktionsmediums nicht für funktionelle Gruppen wie Ester- oder Carbamatgruppen geeignet.

EP-A2 305 115 beschreibt strahlungsaktivierbare 1,3,5-Triazinverbindungen, die mindestens eine halogenhaltige Gruppe CX₃ enthalten und über UV-Belichtung eine radikalische Polymerisation photochemisch auslösen können. Weiterhin können die Triazinverbindungen radikalisch polymerisierbare Gruppen, z. B. Hydroxyethylacrylat, angebunden über eine Urethangruppe, enthalten.

EP-A 359 430 beschreibt ebenfalls halogenhaltige 1,3,5-Triazinverbindungen, die gleichzeitig eine radikalisch polymerisierbare Gruppe enthält. Derartige Verbindungen bilden unter dem Einfluss von Licht Radikale.

Die strahlungsaktivierbaren Halogengruppen In diesen Systemen wirken sich nachteilig auf die UV-Beständigkeit solcher Verbindungen oder Beschichtungen, die solche enthalten, aus und können in der Folge beispielsweise zur Vergilbung von Beschichtungen führen.

JP 2003-280187 beschreibt strahlungshärtbare Verbindungen, in denen Acrylatgruppen über Spacer unter anderem an aromatische Triamine gebunden sind, wobei die Kopplung an die Aminogruppen unter Ausbildung von Carbamatgruppen erfolgt.

In EP-A 366 884 sind 1,3,5-Triazinverbindungen beschrieben, die mindestens zwei vinylterminierte Gruppen und mindestens eine Carbamylgruppe enthalten. Diese 1,3,5-Triazinverbindungen enthalten Reaktionsprodukte von Melamin mit Aldehyden, insbesondere mit Formaldehyd. Neben den Vinylendgruppen enthalten die 1,3,5-Triazinverbindungen Methylol- und/oder alkylierte Methylolpruppen.

Ein ähnliches System ist in EP-A 473 948 beschrieben. Es handelt sich um substituierte 1,3,5-Triazine, die durch Kondensation von Melamin mit Formaldehyd erhalten werden und ethylenisch ungesättigte Gruppen enthalten.

Derartige Systeme sind aufgrund ihrer Aminal- bzw. N,O-Acetalstruktur säureempfindlich.

Aufgabe der vorliegenden Erfindung war es halogenfreie, strahlungshärtbare 1,3,5-Triazincarbamaten und -harnstoffen zur Verfügung zu stellen, die möglichst auch zur Dual-Cure Härtung befähigt sein sollten.

Die Aufgabe wird gelöst durch 1,3,5-Triazincarbamate und -harnstoffe der Formel (I), 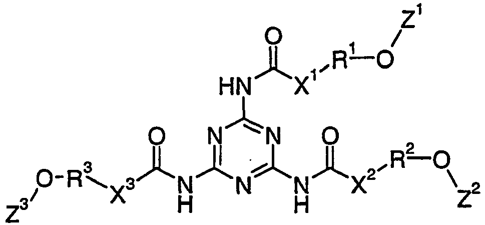 worin
R¹, R² und R³ jeweils unabhängig voneinander einen zweiwertigen organischen Rest,
X¹, X² und X³ jeweils unabhängig voneinander Sauerstoff oder substituierter oder unsubstituierter Stickstoff (NR),
R Wasserstoff oder C₁ - C₂₀-Alkyl und
Z¹, Z² und Z³ jeweils unabhängig voneinander Vinyl, Methacryloyl oder Acryloyl
bedeuten.

Ein weiterer Gegenstand der vorliegenden Erfindung sind 1,3,5-Triazincarbamate und -harnstoffe der Formel (II) 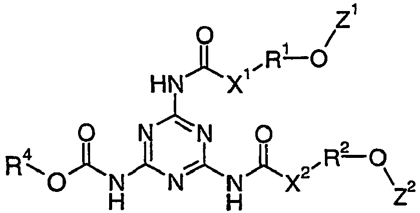 und der Formel (III) 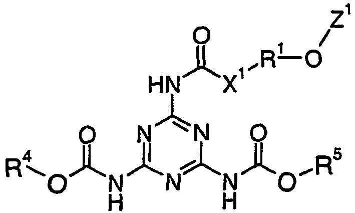 worin
X¹, X², Z¹, Z², R¹ und R² die gleiche Bedeutung haben, wie oben genannt, und
R⁴ und R⁵ jeweils unabhängig voneinander C₁ - C₄-Alkyl
bedeuten.

Ein weiterer Gegenstand der vorliegenden Erfindung sind isocyanatgruppenhaltige 1,3,5-Triazincarbamate und -harnstoffe der Formel (V) 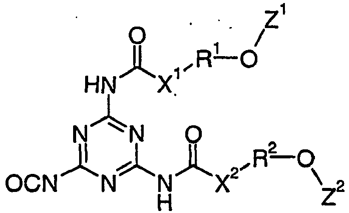 und Formel (VI) 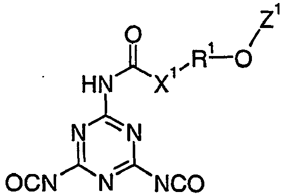 worin
X¹, X², Z¹, Z², R¹ und R² die gleiche Bedeutung haben, wie oben genannt.

Ein weiterer Gegenstand der vorliegenden Erfindung sind Verfahren zur Herstellung dieser Verbindungen.

Erfindungsgemäße strahlungshärtbare 1,3,5-Triazincarbamate und -harnstoffe sind erhältlich durch Umsetzung von Verbindungen der Formel (IV) 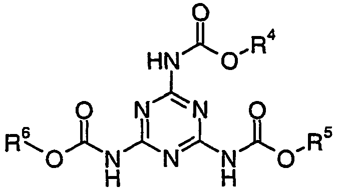 worin
R⁴, R⁵ und R⁶ jeweils unabhängig voneinander C₁ - C₄-Alkyl sein kann,
mit Verbindungen, die eine Hydroxy- oder Aminogruppe und mindestens eine Vinyl-, Methacryloyl- oder Acryloylgruppe aufweisen.

Alternativ sind die Verbindungen der Formel (I), (II) und (III) herstellbar durch Umsetzung von 2,4,6-Triisocyanato-1,3,5-triazin mit Alkoholen oder Aminen der Formel (VII) und im Falle der Verbindungen (II) und (III) gleichzeitiger, vorheriger oder anschließender Umsetzung mit Alkoholen der Formel R⁴OH oder R⁵OH.

Verbindungen der Formeln (V) oder (VI) sind herstellbar durch Umsetzung von 2,4,6-Triisocyanato-1,3,5-triazin mit Alkoholen oder Aminen der Formel (VII).

In den obigen Definitionen bedeuten zweiwertige organische Reste bevorzugt unsubstituiertes oder mit C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₆-C₁₂-Arylen, C₃-C₁₂-Cycloalkylen oder C₁-C₂₀-Alkylen oder durch ein- oder mehrere Sauerstoff- und/oder Schwefelatome und/oder ein oder mehrere substituierte oder unsubstituierte Iminogruppen und/oder durch eine oder mehrere -(CO)-, -O(CO)O-, -(NH)(CO)O-, -O(CO)(NH)-, -O(CO)- oder -(CO)O-Gruppen unterbrochenes C₂-C₂₀-Alkylen.

Darin bedeuten
mit C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₆-C₁₂-Arylen beispielsweise 1,2-, 1,3- oder 1,4-Phenylen, 4,4'-Biphenylen sowie 1',4-Toluylen, 1',1'-Dimethyl-1',4-Toluylen, 2,4-Dimethylphenyl-1',1"-en oder 1',1'-Dimethyl-2,4-Dimethylphenyl-1',1 "-en,
mit C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₃-C₁₂-Cycloalkylen beispielsweise Cyclopropylen, 1,2- oder 1,3-Cyclopentylen, 1,2-, 1,3- oder 1,4-Cyclohexylen, 3,5,5-Trimethyl-1,3-cyclohexylen, Cyclooctylen oder Cyclododecylen,
mit C₁-C₈-Alkyl, C₂-C₈-Alkenyl, Carboxy, Carboxy-C₁-C₈-Alkyl, C₁-C₂₀-Acyl, C₁-C₈-Alkoxy, C₆-C₁₂-Aryl, Hydroxyl oder hydroxysubstituiertem C₁-C₈-Alkyl substituiertes C₁-C₂₀-Alkylen beispielsweise 1,2-Ethylen, 1,2-Propylen, 1,3-Propylen, 1,4-Butylen, 1,6-Hexylen, 1,1-Dimethyl-1,2-ethylen, 2-Methyl-1,3-propylen, 2,2-Dimethyl-1,3-Propylen, 2,2-Dimethyl-1,4-butylen,
durch ein- oder mehrere Sauerstoff- und/oder Schwefelatome und/oder ein oder mehrere substituierte oder unsubstituierte Iminogruppen und/oder durch eine oder mehrere -(CO)-, -O(CO)O-, -(NH)(CO)O-, -O(CO)(NH)-, -O(CO)- oder -(CO)O-Gruppen unterbrochenes C₂-C₂₀-Alkylen beispielsweise 3-Aza-1,5-pentylen, 3-Methyl-3-aza-1,5-pentylen, 3,6-Diaza-1,8-octylen, 3,6,9-Triaza-1,11-undecylen, 3-Oxa-1,5-pentylen, 3,6-Dioxa-1,8-octylen, 3,6,9-Trioxa-1, 11-undecylen,
und C₁ bis C₂₀-Alkyl beispielsweise Methyl, Ethyl, Propyl, Isopropyl, n-Butyl, sec-Butyl, tert.-Butyl, n-Hexyl, n-Heptyl, n-Octyl, n-Decyl, n-Dodecyl, n-Tetradecyl, n-Hexadecyl, n-Octadecyl, n-Eicosyl oder 2-Ethylhexyl.

Bevorzugt für R¹, R² und R³ sind jeweils unabhängig voneinander 1,2-Ethylen, 1,2-Propylen, 1,3-Propylen, 1,4-Butylen, 1,6-Hexylen, 2,2-Dimethyl-1,3-Propylen, 3-Oxa-1,5-pentylen, 3,6-Dioxa-1,8-octylen und 3,6,9-Trioxa-1,11-undecylen, besonders bevorzugt sind 1,2-Ethylen, 1,2-Propylen, 1,3-Propylen, 1,4-Butylen, 1,6-Hexylen und 3-Oxa-1,5-pentylen, ganz besonders bevorzugt sind 1,2-Ethylen, 1,2-Propylen, 1,4-Butylen und 1,6-Hexylen und insbesondere 1,2-Ethylen.

Bevorzugt ist R Wasserstoff oder C₁-C₄-Alkyl, also Methyl, Ethyl, Propyl, Isopropyl, n-Butyl, sec-Butyl, tert.-Butyl, besonders bevorzugt Wassserstoff, Methyl oder n-Butyl, ganz besonders bevorzugt Wasserstoff oder Methyl und insbesondere Wasserstoff.

Beispiele für Verbindungen, die eine Hydroxy- oder Aminogruppe und mindestens eine Vinyl-, Methacryloyl- oder Acryloylgruppe aufweisen, sind solche, die genau eine Hydroxy- oder Aminogruppe, bevorzugt eine Hydroxygruppe, und mindestens eine, beispielsweise 1 bis 10, bevorzugt 1 bis 6, besonders bevorzugt 1 bis 4, ganz besonders bevorzugt 1 bis 3, insbesondere 1 bis 2 und speziell eine Vinyl-, Methacryloyl- oder Acryloylgruppe aufweisen.

Dies können z.B. Vinylether oder (Meth)Acrylsäureester von Polyetherolen, Polyesterolen, Urethanalkoholen, Carbonatalkoholen oder Epoxyalkoholen sein, bevorzugt Polyether(meth)acrylate, Polyesterol(meth)acrylate, Urethan(meth)acrylate oder Epoxy-(meth)acrylate.

Bevorzugt sind solche Verbindungen, wie sie beschrieben sind in EP-A1 54 105, S. 2, Z. 13 bis S. 5, Z. 36 und dort in den Beispielen, in EP-A2 279 303, S. 2, Z. 36 bis S. 3, Z. 34 und dort in den Beispielen, in EP-A1 686621, S. 2, Z. 42 bis S. 4, Z. 47 und dort in den Beispielen, in EP-A1 921 168, Spalte 1, Z. 57 bis Spalte 6, Z. 20 und dort in den Beispielen sowie in DE-A1 33 16 593, S.5, Z. 14 bis S. 10, Z. 4 und dort in den Beispielen.

Besonders bevorzugte Verbindungen, die eine Hydroxy- oder Aminogruppe und mindestens eine Vinyl-, Methacryloyl- oder Acryloylgruppe aufweisen, sind Alkohole oder Amine der Formel (VII)

Z¹-O-R¹-X¹-H, bzw. Z²-O-R²-X²-H oder Z³-O-R³-X³-H.

Darin bedeuten
X¹, X² und X³ jeweils unabhängig voneinander bevorzugt Sauerstoff und
Z¹, Z² und Z³ jeweils unabhängig voneinander bevorzugt Methacryloyl (-(CO)-C(CH₃)=CH₂) oder Acryloyl (-(CO)-C(H)=CH₂), besonders bevorzugt Acryloyl.

Bevorzugte Verbindungen der Formel (VII) sind 2-Hydroxyethyl(meth)acrylat, 2-Hydroxypropyl(meth)acrylat, 4-Hydroxybutyl(meth)acrylat, 6-Hydroxyhexyl(meth)acrylat, Diethylenglykolmono(meth)acrylat, Dipropylenglykolmono(meth)acrylat oder 4-Hydroxybutylvinylether. Weiterhin geeignet sind Trimethylolpropandiacrylat und Pentaerythritdi- und -triacrylat. Besonders bevorzugt sind 2-Hydroxyethyl(meth)acrylat, 2-Hydroxypropyl(meth)acrylat, 4-Hydroxybutyl(meth)acrylat, 6-Hydroxyhexyl(meth)acrylat, Pentaerythrittriacrylat und Diethylenglykolmono(meth)acrylat, ganz besonders bevorzugt sind 2-Hydroxyethyl(meth)acrylat und 2-Hydroxypropyl(meth)acrylat, insbesondere 2-Hydroxyethyl(meth)acrylat und speziell 2-Hydroxyethylacrylat.

Die Verbindung (I) kann vorteilhaft als Vernetzer in strahlungshärtbaren Beschichtungsmassen eingesetzt werden.

Die Verbindungen (II) und (V) können als Vernetzer in strahlungshärtbaren Beschichtungsmassen eingesetzt werden und zusätzlich noch beispielsweise thermisch mit amino- oder hydroxygruppenhaltigen Verbindungen vernetzt werden.

Die Verbindungen (III) und (VI) können als Vernetzer in thermisch härtbaren Beschichtungsmassen zusammen mit mit amino- oder hydroxygruppenhaltigen Verbindungen eingesetzt werden und zusätzlich noch strahlungsgehärtet werden.

Durch den Verzicht auf Halogenatome zeigen die erfindungsgemäßen Verbindungen nicht die aus dem Stand der Technik bekannte UV-Empfindlichkeit und Vergilbungsneigung.

Durch ihre hohe Funktionalitätsdichte können die Verbindungen eine hohe Vernetzung von Beschichtungen erzielen.

Durch ihre über verschiedene Härtungsmechanismen härtbaren funktionellen Gruppen sind besonders die Verbindungen (II), (III), (V) und (VI) vorteilhaft in Dual-Cure-Beschichtungsmassen einsetzbar. Die erfindungsgemäßen Verbindungen enthaltenden Beschichtungen können nach der Härtung eine verbesserte Härte und/oder Kratzfestigkeit aufweisen.

Mit dem Begriff "Dual Cure" beziehungsweise "Multi Cure" ist im Rahmen dieser Schrift ein Härtungsprozeß bezeichnet, der über zwei beziehungsweise mehr als zwei Mechanismen erfolgt und zwar beispielsweise ausgewählt aus strahlungs-, feuchtigkeits-, chemisch, oxidativ und/oder thermisch härtend, bevorzugt ausgewählt aus strahlungs-, feuchtigkeits-, chemisch und/oder thermisch härtend, besonders bevorzugt ausgewählt aus strahlungs-, chemisch und/oder thermisch härtend und ganz besonders bevorzugt strahlungs- und chemisch härtend.

Unter "chemischer Härtung" wird dabei ein Prozeß verstanden, der durch eine chemische Reaktion in der Beschichtungsmasse erfolgt, beispielsweise durch Reaktion von Epoxidgruppen mit Aminen oder bevorzugt Isocyanatgruppen mit Alkoholen oder Aminen oder durch Reaktion von Carbamylgruppen mit Alkoholen.

Die Herstellung der Verbindungen (I), (II) und (III) kann durch eine rein thermische oder bevorzugt durch eine katalysierte Umsetzung von Verbindungen der Formel (IV) mit solchen Verbindungen der Formel (VII) erfolgen.

Bei der thermischen Reaktionsführung wird die Reaktion bei einer Temperatur bis zu 140 °C, bevorzugt bis zu 130 °C durchgeführt.

Da die Temperatur im katalysierten Verfahren niedriger ist als bei der rein thermischen Herstellung sind günstigere Farbzahlen erzielbar.

Die Reste R⁴ - R⁶ sind jeweils unabhängig voneinander abgeleitet von Alkoholen R⁴OH, R⁵OH und R⁶OH, die einen Siedepunkt bei Normaldruck von 120 °C oder weniger aufweisen, bevorzugt von 100 °C oder weniger, besonders bevorzugt von 80 °C oder weniger und ganz besonders bevorzugt von 70 °C oder weniger.

Beispiele für die Reste R⁴, R⁵ und R⁶ sind jeweils unabhängig voneinander Methyl, Ethyl, *iso*-Propyl, n-Propyl, n-Butyl, *iso*-Butyl, *sek-*Butyl*, tert*-Butyl, bevorzugt sind Methyl, Ethyl und n-Butyl, ganz besonders bevorzugt sind Methyl und n-Butyl und insbesondere Methyl.

Die Reste R⁴, R⁵ und R⁶ können gleich oder verschieden sein, bevorzugt handelt es sich um nicht mehr als zwei verschiedene Reste.

Bevorzugte eingesetzte 1,3,5-Triazincarbamate (IV) sind die Methyl-1,3,5-Triazincarbamate, Ethyl-1,3,5-triazincarbamate, n-Butyl-1,3,5-triazincarbamate oder gemischte Methyl-/n-Butyl-1,3,5-Triazincarbamate.

Besonders bevorzugt im erfindungsgemäßen Verfahren sind solche Alkohole bzw. Amine (VII), deren niedrigst siedendes eine Siedepunktsdifferenz von mindestens 20 °C, bevorzugt mindestens 40 und besonders bevorzugt mindestens 60 °C gegenüber dem höchstsiedenden der Alkohole R⁴OH, R⁵OH und R⁶OH aufweisen.

Bei der katalysierten Reaktionsführung ist der Katalysator erfindungsgemäß bevorzugt ausgewählt aus der Gruppe umfassend Zinnverbindungen, Cäsiumsalze, Alkalimetallcarbonate und tertiäre Amine.

Zinnverbindungen sind alle organometallischen Zinnverbindungen, bevorzugt Zinn-(II)-n-octanoat, Zinn-(II)-2-ethyl-1-hexanoat, Zinn-(II)-laurat, Dibutylzinnoxid, Dibutylzinndichlorid, Dibutylzinndiacetat, Dibutylzinndilaurat, Dibutylzinndimaleat oder Dioctylzinndiacetat, besonders bevorzugt sind Zinn(II)-n-octanoat, Zinn-(II)-2-ethyl-I-hexanoat, Zinn-(II)-laurat, Dibutylzinnoxid, Dibutylzinndiacetat, Dibutylzinndilaurat, ganz besonders bevorzugt sind Dibutylzinnoxid, Dibutylzinndiacetat, Dibutylzinndilaurat und insbesondere Dibutylzinndilaurat.

Zinnverbindungen sind jedoch toxikologisch bedenklich und daher erfindungsgemäß weniger bevorzugt, besonders dann, wenn sie in dem Reaktionsgemisch verbleiben. Demgegenüber sind Cäsiumsalze und Alkalimetallcarbonate unbedenklich. Bevorzugte Cäsiumsalze sind dabei solche, in denen folgende Anionen enthalten sind: F⁻, Cl⁻, ClO⁻, ClO₃⁻, ClO₄⁻, Br⁻, J⁻, JO₃⁻, CN⁻, OCN⁻, NO₂⁻, NO₃⁻, HCO₃⁻, CO₃²⁻, S²⁻, SH⁻, HSO₃⁻, SO₃²⁻, HSO₄⁻, SO₄²⁻, S₂O₂²⁻, S₂O₄²⁻, S₂O₅²⁻, S₂O₆²⁻, S₂O₇²⁻, S₂O₈²⁻, H₂PO₂⁻, H₂PO₄⁻, HPO₄²⁻, PO₄³⁻, P₂O₇⁴⁻, (OCₘH₂ₘ₊₁)⁻, (CₘH₂ₘ₋₁O₂)⁻, (CₘH₂ₘ₋₃O₂)⁻ sowie (Cₘ₊₁H₂ₘ₋₂O₄)²⁻, wobei m für die Zahlen 1 bis 20 steht.

Besonders bevorzugt sind dabei Cäsiumcarboxylate, bei denen das Anion den Formeln (CₘH₂ₘ₋₁O₂)⁻ sowie (Cₘ₊₁H₂ₘ₊₂O₄)²⁻ mit m gleich 1 bis 20, gehorcht. Ganz besonders bevorzugte Cäsiumsalze weisen als Anionen Monocarboxylate der allgemeinen Formel (CₘH₂ₘ₋₁O₂)⁻ auf, wobei m für die Zahlen 1 bis 20 steht. Hierbei sind insbesondere zu erwähnen Formiat, Acetat, Propionat, Hexanoat und 2-Ethylhexanoat, ganz besonders bevorzugt ist Cäsium acetat.

Die Cäsiumsalze können dem Ansatz in fester Form, oder gelöster Form zugesetzt werden. Als Lösemittel sind polare, aprotische Lösemittel oder auch protische Lösemittel geeignet. Besonders geeignet sind neben Wasser auch Alkohole; ganz besonders geeignet sind Polyole, wie z.B. Ethan-, Propan-oder Butandiole und Glykolether.

Zur Verbesserung der Löslichkeit der Cäsiumsalze im Reaktionsmedium können diese gegebenenfalls mit Phasentransferkatalysatoren eingesetzt werden. Geeignete Phasentransferkatalysatoren sind beispielsweise Kronenether wie 18-Krone-6 oder Tetraalkylamminumsalze wie Tetrabutylammoniumbromid.

Alkalimetallcarbonate sind beispielsweise Li₂CO₃, Na₂CO₃ und K₂CO₃ sowie die Hydrogencarbonate LiHCO₃, NaHCO₃ und KHCO₃, bevorzugt sind Na₂CO₃ und K₂CO₃ und besonders bevorzugt K₂CO₃.

Tertiäre Amine sind beispielsweise Trioctylamin, Tridodecylamin, Tribenzylamin, N,N,N',N'-Tetramethylethylendiamin, 1-Methylpyrrol, Pyridin, 4-Dimethylaminopyridin, Picolin, N,N'-Dimethylpiperazin, N-Methylmorpholin, N-Methylpiperidin, N-Ethylpiperidin, N,N-Dimethylanilin, N,N-Dimethylbenzylamin, N-Methylimidazol, 1,4-Diazabicyclo[2.2.2]octan, 1,5-Diazabicyclo[4.3.0]non-5-en oder 1,8-Diazabicyclo[5.4.0] undec-7-en.

Bevorzugte Katalysatoren sind Cäsiumsalze und Alkalimetallcarbonate, besonders bevorzugt sind die Cäsiumsalze.

Weiterhin denkbar wären als Katalysatoren Alkoholate (beispielsweise Natrium- oder Kalium Alkoholate von C₁-C₄-Alkylalkoholen, bevorzugt Natrium- und Kalium- -methanolat und -ethanolat), Hydroxide (beispielsweise NaOH, KOH, Ca(OH)₂), Carboxylate (beispielsweise Natrium- oder Kaliumsalze von C₁-C₄-Alkylcarbonsäuren oder ClCH₂COONa), Oxide (beispielsweise CaO, MgO, ZnO, Tl₂O₃, PbO), Phosphine (beispielsweise PPh₃), Zinksalze (ZnCl₂) und Ionentauscher (stark oder schwach alkalische Anionentauscher, wie z.B. DOWEX® MSA-1).

Der Katalysator wird üblicherweise in Mengen 0,001 bis 0,3 mol%, bevorzugt 0,005 bis 0,25, besonders bevorzugt 0,01 bis 0,2 und ganz besonders bevorzugt 0,02 bis 0,1 mol% bezogen auf die Ausgangsverbindung (II) eingesetzt.

Die Reaktion wird erfindungsgemäß bei einer Temperatur von mindestens 40°C, bevorzugt mindestens 50, besonders bevorzugt mindestens 60 und ganz besonders bevorzugt mindestens 70 °C durchgeführt.

Die Reaktionstemperatur liegt bevorzugt oberhalb der Siedetemparatur des abzutrennenden Alkohols R⁴OH, R⁵OH und R⁶OH.

Erfindungsgemäß beträgt die obere Grenze der Temperatur in der Regel nicht mehr als 120 °C, bevorzugt nicht mehr als 110 °C.

Um eine Polymerisation der doppelbindungshaltigen Verbindungen (VII) zu vermindern, wird die Reaktion bevorzugt in Gegenwart von Radikalstabilisatoren durchgeführt. Geeignete Radikalstabilisatoren sind z. B. 4-Methoxyphenol (100-4000 ppm), 2,6-Di-t-Butylhydrochinon (50-1000 ppm), Phenothiazin (10-500 ppm), Triphenylphosphit (50-1000 ppm) sowie 4-Hydroxy-2,2,6,6-tetramethyl-piperidin-N-oxyl (10-500 ppm).

Ein Vorteil der katalysierten Reaktion ist, daß durch die Zugabe des Katalysators bei gleicher oder verkürzter Reaktionszeit und mindestens gleichen Umsätzen unter ansonsten gleichen Bedingungen die Reaktionstemperatur um mindestens 10 °C, bevorzugt mindestens 15 und besonders bevorzugt mindestens 20 °C gegenüber der unkatalysierten Reaktion abgesenkt werden kann.

Die Reaktionszeit ist je nach Substrat unterschiedlich und kann von 15 min bis 12 Stunden betragen, bevorzugt 30 min bis 10 Stunden, besonders bevorzugt 45 min bis 8 Stunden und ganz besonders bevorzugt 1 bis 7 Stunden.

Die Stöchiometrie bzgl. eingesetztem Alkohol bzw. Amin (VII) im Verhältnis zu umzusetzenden Carbamatgruppen in (IV) beträgt in der Regel 0,5 - 1,5 : 1 mol/mol, bevorzugt 0,7 - 1,3 : 1, besonders bevorzugt 0,8 - 1,2 : 1, ganz besonders bevorzugt 0,8 - 1,1 : 1, insbesondere 0,9 - 1:1 und speziell 0,95 - 1,0 : 1 mol/mol.

Je nach gewählter Stöchiometrie zwischen (IV) und (VII) werden unterschiedliche Anteile (I), (II) und (III) im Reaktionsgemisch erhalten.

Wird ein Überschuß an Verbindung (VII) eingesetzt, so kann dieser bevorzugt im Reaktionsgemisch und in der Beschichtungsmasse verbleiben un dkann bei einer Härtung über einen radikalischen oder chemischen Härtungsmechanismus in das Bindemittel eingebaut werden.

Die Reaktion kann in Substanz oder in einem geeigneten Lösungsmittel erfolgen, d.h. einem Lösungsmittel, das nicht mit einem 1,3,5-Triazincarbamat oder -harnstoff reagiert. Dies können beispielsweise sein Aceton, Acetylaceton, Acetoessigester, Ethylacetat, Butylacetat, Ethylenglycoldimethylether, Ethylenglycoldiethylether, Ethylenglycoldi-n-butylether, Diethylenglycoldimethylether, Diethylenglycoldiethylether, Diethylenglycoldi-n-butylether, C₁-C₄-Alkylencarbonate, insbesondere Propylencarbonat , THF, Dioxan, Dimethylformamid, Dimethylsulfoxid, N-Methylpyrrolidon, 1,3-Dioxolan, *iso*-Butyl-methylketon, Ethylmethylketon, Diethylether, tert-Butylmethylether, tert-Butylethylether, n-Pentan, n-Hexan, n-Heptan, Cyclohexan, Methylcyclohexan, Benzol, Toluol, Xylol, Paraffine, Naphta, Mineralöl oder Petroletherfraktionen.

Bevorzugt wird die Reaktion in Substanz durchgeführt.

Die Umsätze, die mit dem genannten Verfahren erzielt werden, betragen in der Regel mindestens 20 %, bevorzugt mindestens 30 %, besonders bevorzugt mindestens 40 und ganz besonders bevorzugt mindestens 60 %.

Die Reaktion kann in einem unter den Reaktionsbedingungen inerten Gas oder Gasgemisch durchgeführt, beispielsweise solche mit einem Sauerstoffgehalt unter 10, bevorzugt unter 8, besonders bevorzugt unter 7 Vol%, bevorzugt sind Stickstoff, Argon, Helium, Stickstoff - Edelgas - Gemische, Kohlenstoffdi- oder -monooxid, besonders bevorzugt ist Stickstoff.

In einer bevorzugten Ausführungsform des erfindungsgemäßen Verfahrens werden die freigesetzten niederen Alkohole R⁴OH, R⁵OH, R⁶OH in geeigneter Weise abgetrennt, wodurch das sich einstellende Reaktionsgleichgewicht zugunsten des Produktes verschoben wird.

Die Abtrennung des niederen Alkohols R⁴OH, R⁵OH, R⁶OH kann beispielsweise erfolgen durch Destillation, Strippen, Vakuum, azeotrope Entfernung, Absorption, Pervaporation und Diffusion über Membranen.

Bevorzugt ist Abdestillieren, gegebenenfalls unter Vakuum, was gegebenenfalls durch Strippen mit einem unter den Reaktionsbedingungen inerten Gas unterstützt werden kann.

Zum Strippen wird ein unter den Reaktionsbedingungen inertes Gas oder Gasgemisch durch das Reaktionsgemisch geleitet, beispielsweise durch Einperlung.

Absorption kann beispielsweise mit Molekularsieben (Porengröße z.B. im Bereich von etwa 3-10 Angström) erfolgen. Diffusion kann beispielsweise mit Hilfe geeigneter semipermeabler Membranen erfolgen.

Die Reaktion kann erfindungsgemäß kontinuierlich, diskontinuierlich oder semikontinuierlich erfolgen, bevorzugt diskontinuierlich oder semikontinuierlich.

Dazu wird in der Regel das Ausgangsmaterial der Formel (IV) vorgelegt und auf die gewünschte Reaktionstemperatur gebracht.

Vor oder nach Erreichen der gewünschten Reaktionstemperatur kann der Katalysator zumindest teilweise zugegeben werden und der Alkohol/das Amin (VII) vollständig, portionsweise oder kontinuierlich zugegeben werden. Falls der Katalysator noch nicht vollständig zugegeben worden ist, kann dieser ebenfalls portionsweise nachgegeben werden.

Es kann vorteilhaft sein, die Reaktionstemperatur im Verlauf der Reaktion zu steigern, beispielsweise um mindestens 10 °C, bevorzugt um mindestens 15 und besonders bevorzugt um mindestens 20 °C gegenüber der Temperatur zu Beginn der Reaktion.

Der Verlauf der Reaktion kann beispielsweise verfolgt werden, in dem man die Menge des freigesetzten Alkohols R⁴OH, R⁵OH und R⁶OH verfolgt und bei dem gewünschten Umsatz abbricht.

Die Reaktion kann beispielsweise durch Herunterkühlen oder durch direktes Abkühlen mit einem Lösungsmittel gestoppt werden.

Bevorzugt wird die Reaktion in einem rückvermischten Reaktionskessel durchgeführt, der beispielsweise durch Rühren, Eindüsen oder durch einen Umpumpkreislauf durchmischt werden kann.

Die Temperatureinstellung kann entweder über die Reaktorwände erfolgen oder durch einen im Umpumpkreislauf befindlichen Wärmetauscher.

Wird der freigesetzte niedere Alkohol R⁴OH, R⁵OH und R⁶OH durch Destillation und/oder Strippen abgetrennt, so kann dem Reaktor eine Packungs- oder Bodenkolonne aufgesetzt sein, für die 2 bis 10 theroretische Böden in der Regel ausreichend sind. Zur Unterstützung der Abtrennung des niederen Alkohols kann ein leichtes Vakuum angelegt werden, beispielsweise kann die Reaktion bei einem Druck von 200 hPa bis Normaldruck, bevorzugt 300 hPa bis Normaldruck, besonders bevorzugt 500 hPa bis Normaldruck, ganz besonders bevorzugt bei 800 hPa bis Normaldruck und insbesondere bei Normaldruck durchgeführt werden.

Nach Beendigung der Reaktion kann das Reaktionsgemisch noch einer Wäsche und/oder Entfärbung unterzogen werden.

Zur Wäsche wird das Reaktionsgemisch in einem Waschapparat mit einer Waschflüssigkeit, beispielsweise Wasser oder einer 5 - 30 Gew%-igen, bevorzugt 5 - 20, besonders bevorzugt 5 - 15 Gew%-igen Kochsalz-, Kaliumchlorid-, Ammoniumchlorid-, Natriumsulfat- oder Ammoniumsulfatlösung, bevorzugt Wasser oder Kochsalzlösung, behandelt.

Die Wäsche kann beispielsweise in einem Rührbehälter oder in anderen herkömmlichen Apparaturen, z.B. in einer Kolonne oder Mixer-Settler-Apparatur, durchgeführt werden.

Falls erforderlich kann das Reaktionsgemisch einer Entfärbung, beispielsweise durch Behandlung mit Aktivkohle oder Metalloxiden, wie z.B. Aluminiumoxid, Siliciumoxid, Magnesiumoxid, Zirkonoxid, Boroxid oder Gemischen davon, in Mengen von beispielsweise 0,1 - 50 Gew%, bevorzugt 0,5 bis 25 Gew%, besonders bevorzugt 1 - 10 Gew% bei Temperaturen von beispielsweise 10 bis 100 °C, bevorzugt 20 bis 80 °C und besonders bevorzugt 30 bis 60 °C unterworfen werden.

Dies kann durch Zugabe des pulver- oder granulatförmigen Entfärbungsmittels zum Reaktionsgemisch und nachfolgender Filtration oder durch Überleiten des Reaktionsgemisches über eine Schüttung des Entfärbungsmittels in Form beliebiger, geeigneter Formkörper erfolgen.

In der Regel ist das unter den genannten Reaktionsbedingungen erhältliche Reaktionsgemisch wie folgt zusammengesetzt:
(I) 5 - 80 Gew%,
(II) 10 - 60 Gew%,
(III) 10 - 40 Gew%,
(IV) 0 - 20 Gew%,
(VII) 0 - 10 Gew%,
mit der Maßgabe, dass die Summe 100 Gw.,-% beträgt,
sowie gegebenenfalls 2-Amino-4,6-biscarbamoyl-1,3,5-triazin oder 2,4-Diamino-6-carbamoyl-1,3,5-triazin als Nebenprodukte der Reaktion in Anteilen unter 10 Gewichtsprozent. Derartige Nebenprodukte können beispielsweise entstehen durch Hydrolyse von Verbindungen der Formeln (I) bis (VI), etwa während der erfindungsgemäßen Umsetzung oder durch enthaltenes Wasser in der Beschichtungsmasse.

Demzufolge sind auch Verbindungen der Formel (IX) 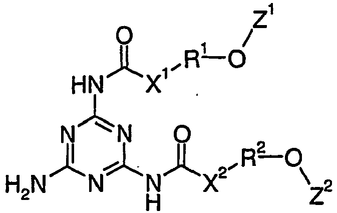 und (X) 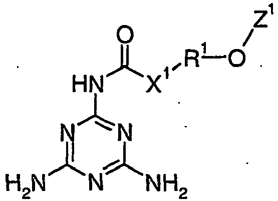 worin
R¹, R², X¹, X², Z¹ und Z² die oben angeführten Bedeutungen haben,

Gegenstand der vorliegenden Erfindung.

Verbindungen der Formel (IX) und (X) sind ebenfalls radikalisch einpolymerisierbar und wirken im Fall der Verbindung (IX) vernetzend.

Die Herstellung der Verbindungen (I), (V) und (VI) aus 2,4,6-Triisocyanato-[1,3,5]triazin kann prinzipiell genauso erfolgen, wie oben von der Verbindung (IV) ausgehend beschrieben ist, jedoch mit folgenden Unterschieden:

Die Stöchiometrie bzgl. eingesetztem Alkohol bzw. Amin (VII) im Verhältnis zu umzusetzenden Isocyanatgruppen in 2,4,6-Triisocyanato-[1,3,5]triazin beträgt in der Regel 0,5 - 1,2 : 1 mol/mol, bevorzugt 0,7 - 1,1 : 1, besonders bevorzugt 0,8 - 1,1 : 1, ganz besonders bevorzugt 0,9 - 1,1 : 1, insbesondere 0,9 - 1:1 und speziell 0,95 - 1,0 : 1 mol/mol.

In der Regel wird die Reaktion bei Temperaturen zwischen 5 und 100 °C, bevorzugt zwischen 20 bis 90 °C und besonders bevorzugt zwischen 40 und 80°C und insbesondere zwischen 60 und 80 °C durchgeführt.

Bevorzugt wird dabei unter wasserfreien Bedingungen gearbeitet.

Wasserfrei bedeutet dabei, daß der Wassergehalt im Reaktionssystem nicht mehr als 5 Gew.% beträgt, bevorzugt nicht mehr als 3 Gew.% und besonders bevorzugt nicht mehr als 1 Gew.-%.

Zur Beschleunigung der Reaktion der Diisocyanate können die üblichen Katalysatoren mitverwendet werden. Dafür kommen prinzipiell alle in der Polyurethanchemie üblicherweise verwendeten Katalysatoren in Betracht.

Diese sind beispielsweise organische Amine, insbesondere tertiäre aliphatische, cycloaliphatische oder aromatische Amine, und/oder Lewis-saure organische Metallverbindungen. Als Lewis-saure organische Metallverbindungen kommen z.B. die oben genannten Zinnverbindungen in Frage, wie beispielsweise Zinn-(II)-salze von organischen Carbonsäuren, z.B. Zinn(II)-acetat, Zinn(II)-octoat, Zinn(II)-ethylhexoat und Zinn(II)-laurat und die Dialkylzinn(IV)-salze von organischen Carbonsäuren, z.B. Dimethylzinn-diacetat, Dibutylzinn-diacetat, Dibutylzinn-dibutyrat, Dibutylzinn-bis(2-ethylhexanoat), Dibutylzinn-dilaurat, Dibutylzinn-maleat, Dioctylzinn-dilaurat und Dioctylzinn-diacetat. Auch Metallkomplexe wie Acetylacetonate des Eisens, Titans, Aluminiums, Zirkons, Mangans, Nickels und Cobalts sind möglich. Weitere Metallkatalysatoren werden von Blank et al. in Progress in Organic Coatings, 1999, Vol. 35, Seiten 19-29 beschrieben.

Bevorzugte Lewis-saure organische Metallverbindungen sind Dimethylzinn-diacetat, Dibutylzinn-dibutyrat, Dibutylzinn-bis(2-ethylhexanoat), Dibutylzinn-dilaurat, Dioctylzinn-dilaurat, Zirkon-Acetylacetonat und Zirkon-2,2,6,6-tetramethyl-3,5-heptandionat.

Auch Wismut-und Cobaltkatalysatoren sowie Cäsiumsalze können als Katalysatoren eingesetzt werden. Als Cäsiumsalze kommen dabei die oben genannten Verbindungen in Betracht, bevorzugt Cäsiumacetat.

Aufgrund der Reaktivität der Isocyanatgruppen in den Verbindungen (V) und (VI) wird in der Regel auf eine Wäsche verzichtet.

Die erfindungsgemäßen 1,3,5-Triazincarbamate und -harnstoffe der Formeln (I), (II), (III), (V) und (VI) können verwendet werden zur Beschichtung von verschiedenen Substraten, wie z. B. Holz, Holzfurnier, Papier, Pappe, Karton, Textil, Leder, Vlies, Kunststoffoberflächen, Glas, Keramik, mineralische Baustoffe, Metalle oder beschichtete Metalle.

Bei einer Verwendung in Beschichtungsmitteln können die erfindungsgemäßen 1,3,5-Triazincarbamate und -harnstoffe insbesondere in Grundierungen, Füllern, pigmentierten Decklacken und Klarlacken im Bereich Autoreparatur- oder Großfahrzeuglackierung eingesetzt werden. Besonders geeignet sind solche Beschichtungsmittel für Anwendungen, in denen eine besonders hohe Applikationssicherheit, Außenwitterungsbeständigkeit, Optik, Lösemittel-, Chemikalien- und Wasserfestigkeit gefordert werden, wie in der Autoreparatur- und Großfahrzeuglackierung.

Ein weiterer Gegenstand der vorliegenden Erfindung sind strahlungshärtbare Beschichtungsmassen, enthaltend
- mindestens eine Verbindung der Formel (I), (II), (III), (V) oder (VI),
- gegebenenfalls eine Verbindung mit einer oder mehr als einer radikalisch polymerisierbaren Doppelbindung,
- gegebenenfalls mindestens einen Photoinitiator und
- gegebenenfalls weitere lacktypische Additive.

Die erfindungsgemäßen Verbindungen (I), (II), (III), (V) oder (VI) können als alleiniges Bindemittel oder in Kombination mit einem weiteren radikalisch polymerisierbaren Verbindung verwendet werden.

Verbindungen mit einer oder mehr als einer radikalisch polymerisierbaren Doppelbindung sind beispielsweise solche Verbindungen, die 1 bis 6, bevorzugt 1 bis 4 und besonders bevorzugt 1 bis 3 radikalisch polymerisationsfähige Gruppen aufweisen.

Radikalisch polymerisationsfähige Gruppen sind beispielsweise Vinylether- oder (Meth)Acrylat-Gruppen, bevorzugt (Meth)Acrylat-Gruppen und besonders bevorzugt Acrylat-Gruppen.

Radikalisch polymerisationsfähige Verbindungen werden häufig unterteilt in monofunktionelle (Verbindung mit einer radikalisch polymerisierbaren Doppelbindung) und multifunktionelle (Verbindung mit mehr als einer radikalisch polymerisierbaren Doppelbindung), polymerisationsfähige Verbindungen.

Monofunktionelle, polymerisationsfähige Verbindungen sind solche mit genau einer radikalisch polymerisationsfähigen Gruppe, multifunktionelle, polymerisationsfähige Verbindungen solche mit mehr als einer, bevorzugt mit mindestens zwei radikalisch polymerisationsfähigen Gruppen.

Monofunktionelle, polymerisationsfähige Verbindungen sind beispielsweise Ester der (Meth)acrylsäure mit Alkoholen, die 1 bis 20 C-Atome aufweisen, z.B. (Meth)acrylsäuremethylester, (Meth)acrylsäureethylester, (Meth)acrylsäurebutylester, (Meth)acrylsäure-2-ethylhexylester, 2-Hydroxyethyl(meth)acrylat, 2-Hydroxypropyl(meth)acrylat, 4-Hydroxybutyl(meth)acrylat, Dihydrodicyclopentadienylacrylat, Vinylaromatische Verbindungen, z.B. Styrol, Divinylbenzol, α,β-ungesättigte Nitrile, z.B. Acrylnitril, Methacrylnitril, α,β-ungesättigte Aldehyde, z.B. Acrolein, Methacrolein, Vinylester, z.B. Vinylacetat, Vinylpropionat, halogenierte ethylenisch ungesättigte Verbindungen, z.B. Vinylchlorid, Vinylidenchlorid, konjugierte ungesättigte Verbindungen, z.B. Butadien, Isopren, Chloropren, einfach ungesättigte Verbindungen, z.B. Ethylen, Propylen, 1-Buten, 2-Buten, iso-Buten, cyclische einfach ungesättigte Verbindungen, z.B. Cyclopenten, Cyclohexen, Cyclododecen, N-Vinylformamid, Allylessigsäure, Vinylessigsäure, monoethylenisch ungesättigten Carbonsäuren mit 3 bis 8 C-Atomen sowie deren wasserlöslichen Alkalimetall-, Erdalkalimetall- oder Ammoniumsalze wie beispielsweise: Acrylsäure, Methacrylsäure, Dimethylacrylsäure, Ethacrylsäure, Maleinsäure, Citraconsäure, Methylenmalonsäure, Crotonsäure, Fumarsäure, Mesaconsäure und Itaconsäure, Maleinsäure, N-Vinylpyrrolidon, N-Vinyllactame, wie z.B. N-Vinylcaprolactam, N-Vinyl-N-Alkyl-carbonsäureamide oder N-Vinyl-carbonsäureamide, wie z. B. N-Vinylacetamid, N-Vinyl-N-methylformamid und N-Vinyl-N-methylacetamid oder Vinylether, z.B. Methylvinylether, Ethylvinylether, *n*-Propylvinylether, *iso*-Propylvinylether, *n*-Butylvinylether, *sek*-Butylvinylether, *iso*-Butylvinylether, *tert*-Butylvinylether, 4-Hydroxybutylvinylether, sowie Gemische davon.

Unter diesen bevorzugt sind die Ester der (Meth)Acrylsäure, besonders bevorzugt sind (Meth)acrylsäuremethylester, (Meth)acrylsäureethylester, (Meth)acrylsäure-n-butylester, (Meth)acrylsäure-2-ethylhexylester und 2-Hydroxyethylacrylat, ganz besonders bevorzugt sind (Meth)acrylsäure-n-butylester, (Meth)acrylsäure-2-ethylhexylester und 2-Hydroxyethylacrylat und insbesondere 2-Hydroxyethylacrylat.

(Meth)Acrylsäure steht in dieser Schrift für Methacrylsäure und Acrylsäure, bevorzugt für Acrylsäure.

Multifunktionelle, polymerisationsfähige Verbindungen sind bevorzugt multifunktionelle (Meth)acrylate, die mehr als 1, bevorzugt 2 - 10, besonders bevorzugt 2 - 6, ganz besonders bevorzugt 2 - 4 und insbesondere 2 - 3 (Meth)acrylatgruppen, bevorzugt Acrylatgruppen tragen.

Dies können beispielsweise Ester der (Meth)acrylsäure mit entsprechend mindestens zweiwertigen Polyalkoholen sein.

Derartige geeignete Polyalkohole sind beispielsweise mindestens zweiwertige Polyole, Polyether- oder Polyesterole oder Polyacrylatpolyole mit einer mittleren OH-Funktionalität von mindestens 2, bevorzugt 3 bis 10.

Beispiele für multifunktionelle, polymerisationsfähige Verbindungen sind Ethylenglykoldiacrylat, 1,2-Propandioldiacrylat, 1,3-Propandioldiacrylat, 1,4-Butandioldiacrylat, 1,3-Butandioldiacrylat, 1,5-Pentandioldiacrylat, 1,6-Hexandioldiacrylat, 1,8-Octandioldiacrylat, Neopentylglykoldiacrylat, 1,1-, 1,2-, 1,3- und 1,4-Cyclohexandimethanoldiacrylat, 1,2-, 1,3- oder 1,4-Cyclohexandioldiacrylat, Trimethylolpropantriacrylat, Ditrimethylolpropanpenta- oder -hexaacrylat, Pentaerythrittri- oder tetraacrylat, Glycerindi- oder -triacrylat, sowie Di- und Polyacrylate von Zuckeralkoholen, wie beispielsweise Sorbit, Mannit, Diglycerol, Threit, Erythrit, Adonit (Ribit), Arabit (Lyxit), Xylit, Dulcit (Galactit), Maltit oder Isomalt, oder von Polyesterpolyolen, Polyetherolen, Poly-THF mit einer Molmasse zwischen 162 und 2000, Poly-1,3-Propandiol mit einer Molmasse zwischen 134 und 1178, Polyethylenglykol mit einer Molmasse zwischen 106 und 898, sowie Epoxy(meth)acrylate, Urethan(meth)acrylate oder Polycarbonat(meth)acrylate.

Weitere Beispiele sind (Meth)Acrylate von Verbindungen der Formel (VIIIa) bis (Vlllc), 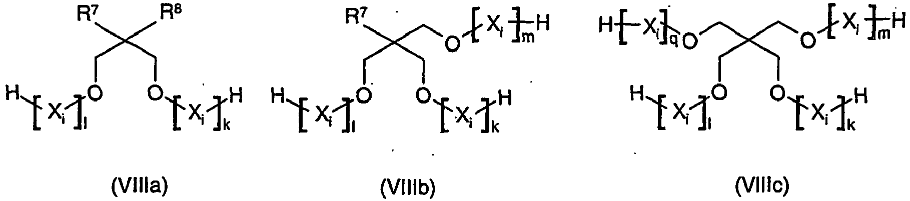 worin
R⁷ und R⁸ unabhängig voneinander Wasserstoff oder gegebenenfalls durch Aryl, Alkyl, Aryloxy, Alkyloxy, Heteroatome und/oder Heterocyclen substituiertes C₁ - C₁₈-Alkyl,
k, l, m, q unabhängig voneinander je für eine ganze Zahl von 1 bis 10, bevorzugt 1 bis 5 und besonders bevorzugt 1 bis 3 steht und
jedes Xᵢ für i =1 bis k, 1 bis I, 1 bis m und 1 bis q unabhängig voneinander ausgewählt sein kann aus der Gruppe -CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CHVin-O-, -CHVin-CH₂-O-, -CH₂-CHPh-O- und -CHPh-CH₂-O-, bevorzugt aus der Gruppe -CH₂-CH₂-O-, -CH₂-CH(CH₃)-O- und -CH(CH₃)-CH₂-O-, und besonders bevorzugt -CH₂-CH₂-O-,
worin Ph für Phenyl und Vin für Vinyl steht.

Darin bedeuten gegebenenfalls durch Aryl, Alkyl, Aryloxy, Alkyloxy, Heteroatome und/oder Heterocyclen substituiertes C₁ - C₁₈-Alkyl beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Hetadecyl, Octadecyl, 1,1-Dimethylpropyl, 1,1-Dimethylbutyl, 1,1,3,3-Tetramethylbutyl, bevorzugt Methyl, Ethyl oder n-Propyl, ganz besonders bevorzugt Methyl oder Ethyl.

Bevorzugt handelt es sich dabei um (Meth)Acrylate von ein- bis zwanzigfach und besonders bevorzugt drei- bis zehnfach ethoxyliertem, propoxyliertem oder gemischt ethoxyliertem und propoxyliertem und insbesondere ausschließlich ethoxyliertem Neopentylglykol, Trimethylolpropan, Trimethylolethan oder Pentaerythrit.

Bevorzugte multifunktionelle, polymerisationsfähige Verbindungen sind Ethylenglykoldiacrylat, 1,2-Propandioldiacrylat, 1,3-Propandioldiacrylat, 1,4-Butandioldiacrylat, 1,6-Hexandioldiacrylat, Trimethylolpropantriacrylat, Pentaerythrittetraacrylat, Polyestepolyolenacrylate, Polyetherolacrylate und Triacrylat von ein- bis zwanzigfach alkoxyliertem, besonders bevorzugt ethoxyliertem Trimethylolpropan.

Ganz besonders bevorzugte multifunktionelle, polymerisationsfähige Verbindungen sind 1,4-Butandioldiacrylat, 1,6-Hexandioldiacrylat, Trimethylolpropantriacrylat, Pentaerythrittetraacrylat und Triacrylat von ein- bis zwanzigfach ethoxyliertem Trimethylolpropan.

Polyesterpolyole, sind z.B. aus Ullmanns Encyklopädie der technischen Chemie, 4. Auflage, Band 19, S. 62 bis 65 bekannt. Bevorzugt werden Polyesterpolyole eingesetzt, die durch Umsetzung von zweiwertigen Alkoholen mit zweiwertigen Carbonsäuren erhalten werden. Anstelle der freien Polycarbonsäuren können auch die entsprechenden Polycarbonsäureanhydride oder entsprechende Polycarbonsäureester von niederen Alkoholen oder deren Gemische zur Herstellung der Polyesterpolyole verwendet werden. Die Polycarbonsäuren können aliphatisch, cycloaliphatisch, araliphatisch, aromatisch oder heterocyclisch sein und gegebenenfalls, z.B. durch Halogenatome, substituiert und/oder ungesättigt sein. Als Beispiele hierfür seien genannt:

Oxalsäure, Maleinsäure, Fumarsäure, Bernsteinsäure, Glutarsäure, Adipinsäure, Sebacinsäure, Dodekandisäure, o-Phthalsäure, Isophthalsäure, Terephthalsäure, Trimellithsäure, Azelainsäure, 1,4-Cyclohexandicarbonsäure oder Tetrahydrophthalsäure, Korksäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäureanhydrid, dimere Fettsäuren, deren Isomere und Hydrierungsprodukte sowie veresterbare Derivate, wie Anhydride oder Dialkylester, beispielsweise C₁-C₄-Alkylester, bevorzugt Methyl-, Ethyl- oder n-Butylester, der genannten Säuren eingesetzt werden. Bevorzugt sind Dicarbonsäuren der allgemeinen Formel HOOC-(CH₂)_{y}-COOH, wobei y eine Zahl von 1 bis 20, bevorzugt eine gerade Zahl von 2 bis 20 ist, besonders bevorzugt Bernsteinsäure, Adipinsäure, Sebacinsäure und Dodecandicarbonsäure.

Als mehrwertige Alkohole kommen zur Herstellung der Polyesterole in Betracht 1,2-Propandiol, Ethylenglykol, 2,2-Dimethyl-1,2-Ethandiol, 1,3-Propandiol, 1,2-Butandiol, 1,3-Butandiol, 1,4-Butandiol, 3-Methylpentan-1,5-diol, 2-Ethylhexan-1,3-diol, 2,4-Diethyloctan-1,3-diol, 1,6-Hexandiol, Poly-THF mit einer Molmasse zwischen 162 und 2000, Poly-1,3-propandiol mit einer Molmasse zwischen 134 und 1178, Poly-1,2-propandiol mit einer Molmasse zwischen 134 und 898, Polyethylenglykol mit einer Molmasse zwischen 106 und 458, Neopentylglykol, Hydroxypivalinsäureneopentylglykolester, 2-Ethyl-1,3-Propandiol, 2-Methyl-1,3-Propandiol, 2,2-Bis(4-hydroxycyclohexyl)propan, 1,1-, 1,2-, 1,3- und 1,4-Cyclohexandimethanol, 1,2-, 1,3- oder 1,4-Cyclohexandiol, Trimethylolbutan, Trimethylolpropan, Trimethylolethan, Pentaerythrit, Glycerin, Ditrimethylolpropan, Dipentaerythrit, Sorbit, Mannit, Diglycerol, Threit, Erythrit, Adonit (Ribit), Arabit (Lyxit), Xylit, Dulcit (Galactit), Maltit oder Isomalt, die gegebenenfalls wie oben beschrieben alkoxyliert sein können.

Bevorzugt sind Alkohole der allgemeinen Formel HO-(CH₂)ₓ-OH, wobei x eine Zahl von 1 bis 20, bevorzugt eine gerade Zahl von 2 bis 20 ist. Bevorzugt sind Ethylenglycol, Butan-1,4-diol, Hexan-1,6-diol, Octan-1,8-diol und Dodecan-1,12- diol. Weiterhin bevorzugt ist Neopentylglykol.

Ferner kommen auch Polycarbonat-Diole, wie sie z.B. durch Umsetzung von Phosgen mit einem Überschuß von den als Aufbaukomponenten für die Polyesterpolyole genannten niedermolekularen Alkohole erhalten werden können, in Betracht.

Geeignet sind auch Polyesterdiole auf Lacton-Basis, wobei es sich um Homo- oder Mischpolymerisate von Lactonen, bevorzugt um endständige Hydroxylgruppen aufweisende Anlagerungsprodukte von Lactonen an geeignete difunktionelle Startermoleküle handelt. Als Lactone kommen bevorzugt solche in Betracht, die sich von Verbindungen der allgemeinen Formel HO-(CH₂)₂-COOH ableiten, wobei z eine Zahl von 1 bis 20 ist und ein H-Atom einer Methyleneinheit auch durch einen C₁- bis C₄-Alkylrest substituiert sein kann. Beispiele sind ε-Caprolacton, β-Propiolacton, gamma-Butyrolacton und/oder Methyl-ε-caprolacton, 4-Hydroxybenzoesäure, 6-Hydroxy-2-naphthalinsäure oder Pivalolacton sowie deren Gemische. Geeignete Starterkomponenten sind z.B. die vorstehend als Aufbaukomponente für die Polyesterpolyole genannten niedermolekularen zweiwertigen Alkohole. Die entsprechenden Polymerisate des ε-Caprolactons sind besonders bevorzugt. Auch niedere Polyesterdiole oder Polyetherdiole können als Starter zur Herstellung der Lacton-Polymerisate eingesetzt sein. Anstelle der Polymerisate von Lactonen können auch die entsprechenden, chemisch äquivalenten Polykondensate der den Lactonen entsprechenden Hydroxycarbonsäuren, eingesetzt werden. Weiterhin kann es sich bei der multifunktionellen, polymerisationsfähigen Verbindung um Urethan(meth)acrylate, Epoxy(meth)acrylate oder Carbonat(meth)acrylate handeln.

Urethan(meth)acrylate sind z.B. erhältlich durch Umsetzung von Polyisocyanaten mit Hydroxyalkyl(meth)acrylaten oder -vinylethern und gegebenenfalls Kettenverlängerungsmitteln wie Diolen, Polyolen, Diaminen, Polyaminen oder Dithiolen oder Polythiolen. In Wasser ohne Zusatz von Emulgatoren dispergierbare Urethan(meth)acrylate enthalten zusätzlich noch ionische und/oder nichtionische hydrophile Gruppen, welche z.B. durch Aufbaukomponenten wie Hydroxycarbonsäuren ins Urethan eingebracht werden.

Die verwendbaren Polyurethane enthalten als Aufbaukomponenten im wesentlichen:
(a) mindestens ein organisches aliphatisches, aromatisches oder cycloaliphatisches Di- oder Polyisocyanat,
(b) mindestens eine Verbindung mit mindestens einer gegenüber Isocyanat reaktiven Gruppe und mindestens einer radikalisch polymerisierbaren ungesättigten Gruppe und
(c) gegebenenfalls mindestens eine Verbindung mit mindestens zwei gegenüber Isocyanat reaktiven Gruppen.

Als Komponente (a) kommen beispielsweise aliphatische, aromatische und cycloaliphatische Di- und Polyisocyanate mit einer NCO Funktionalität von mindestens 1,8, bevorzugt 1,8 bis 5 und besonders bevorzugt 2 bis 4 in Frage, sowie deren Isocyanurate, Biurete, Allophanate und Uretdione.

Bei den Diisocyanaten handelt es sich bevorzugt um Isocyanate mit 4 bis 20 C-Atomen. Beispiele für übliche Diisocyanate sind aliphatische Diisocyanate wie Tetramethylendiisocyanat, Hexamethylendiisocyanat(1,6-Diisocyanatohexan), Octamethylendiisocyanat, Decamethylendiisocyanat, Dodecamethylendiisocyanat, Tetradecamethylendiisocyanat, Derivate des Lysindiisocyanates, Tetramethylxylylendiisocyanat, Trimethylhexandiisocyanat oder Tetramethylhexandiisocyanat, cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5- trimethyl-5-(isocyanatomethyl)cyclohexan (Isophorondiisocyanat), 1,3- oder 1,4-Bis(isocyanatomethyl)cyclohexan oder 2,4-, oder 2,6-Diisocyanato-1-methylcyclohexan sowie aromatische Diisocyanate wie 2,4- oder 2,6-Toluylendiisocyanat und deren Isomerengemische, m- oder p-Xylylendiisocyanat, 2,4'- oder 4,4'-Diisocyanatodiphenylmethan und deren Isomerengemische, 1,3- oder 1,4-Phenylendiisocyanat, 1-Chlor-2,4-phenylendiisocyanat, 1,5-Naphthylendiisocyanat, Diphenylen-4,4'-diisocyanat, 4,4'-Diisocyanato-3,3'-dimethyldiphenyl, 3-Methyldiphenylmethan-4,4'-diisocyanat, Tetramethylxylylendiisocyanat, 1,4-Diisocyanatobenzol oder Diphenylether-4,4'-diisocyanat.

Es können auch Gemische der genannten Diisocyanate vorliegen.

Bevorzugt sind Hexamethylendiisocyanat, 1,3-Bis(isocyanatomethyl)cyclohexan, Isophorondiisocyanat und Di(isocyanatocyclohexyl)methan.

Als Polyisocyanate kommen Isocyanuratgruppen aufweisende Polyisocyanate, Uretdiondiisocyanate, Biuretgruppen aufweisende Polyisocyanate, Urethan- oder Allophanatgruppen aufweisende Polyisocyanate, Oxadiazintriongruppen enthaltende Polyisocyanate, Uretonimin-modifizierte Polyisocyanate von geradlinigen oder verzweigten C₄-C₂₀-Alkylendiisocyanaten, cycloaliphatischen Diisocyanaten mit insgesamt 6 bis 20 C-Atomen oder aromatischen Diisocyanaten mit insgesamt 8 bis 20 C-Atomen oder deren Gemische in Betracht.

Die einsetzbaren Di- und Polyisocyanate haben bevorzugt einen Gehalt an Isocyanatgruppen (berechnet als NCO, Molekulargewicht = 42) von 10 bis 60 Gew% bezogen auf das Di- und Polyisocyanat(gemisch), bevorzugt 15 bis 60 Gew% und besonders bevorzugt 20 bis 55 Gew%.

Bevorzugt sind aliphatische bzw. cycloaliphatische Di- und Polyisocyanate, z.B. die vorstehend genannten aliphatischen bzw. cycloaliphatischen Diisocyanate, oder deren Mischungen.

Weiterhin bevorzugt sind
1) Isocyanuratgruppen aufweisende Polyisocyanate von aromatischen, aliphatischen und/oder cycloaliphatischen Diisocyanaten. Besonders bevorzugt sind hierbei die entsprechenden aliphatischen und/oder cycloaliphatischen Isocyanato-Isocyanurate und insbesondere die auf Basis von Hexamethylendiisocyanat und Isophorondiisocyanat. Bei den dabei vorliegenden Isocyanurate handelt es sich insbesondere um Tris-isocyanatoalkyl- bzw. Tris-isocyanatocycloalkyl-Isocyanurate, welche cyclische Trimere der Diisocyanate darstellen, oder um Gemische mit ihren höheren, mehr als einen Isocyanuratring aufweisenden Homologen. Die Isocyanato-Isocyanurate haben im allgemeinen einen NCO-Gehalt von 10 bis 30 Gew.-%, insbesondere 15 bis 25 Gew.-% und eine mittlere NCO-Funktionalität von 3 bis 4,5.
2) Uretdiondiisocyanate mit aromatisch, aliphatisch und/oder cycloaliphatisch gebundenen Isocyanatgruppen, vorzugsweise aliphatisch und/oder cycloaliphatisch gebundenen und insbesondere die von Hexamethylendiisocyanat oder Isophorondiisocyanat abgeleiteten. Bei Uretdiondiisocyanaten handelt es sich um cyclische Dimerisierungsprodukte von Diisocyanaten.
   Die Uretdiondiisocyanate können in den Zubereitungen als alleinige Komponente oder im Gemisch mit anderen Polyisocyanaten, insbesondere den unter 1) genannten, eingesetzt werden.
3) Biuretgruppen aufweisende Polyisocyanate mit aromatisch, cycloaliphatisch oder aliphatisch gebundenen, bevorzugt cycloaliphatisch oder aliphatisch gebundenen Isocyanatgruppen, insbesondere Tris(6-isocyanatohexyl)biuret oder dessen Gemische mit seinen höheren Homologen. Diese Biuretgruppen aufweisenden Polyisocyanate weisen im allgemeinen einen NCO-Gehalt von 18 bis 22 Gew.-% und eine mittlere NCO-Funktionalität von 3 bis 4,5 auf.
4) Urethan- und/oder Allophanatgruppen aufweisende Polyisocyanate mit aromatisch, aliphatisch oder cycloaliphatisch gebundenen, bevorzugt aliphatisch oder cycloaliphatisch gebundenen Isocyanatgruppen, wie sie beispielsweise durch Umsetzung von überschüssigen Mengen an Hexamethylendiisocyanat oder an Isophorondiisocyanat mit mehrwertigen Alkoholen wie z.B. Trimethylolpropan, Neopentylglykol, Pentaerythrit, 1,4-Butandiol, 1,6-Hexandiol, 1,3-Propandiol, Ethylenglykol, Diethylenglykol, Glycerin, 1,2-Dihydroxypropan oder deren Gemische erhalten werden können. Diese Urethan- und/oder Allophanatgruppen aufweisenden Polyisocyanate haben im allgemeinen einen NCO-Gehalt von 12 bis 20 Gew.% und eine mittlere NCO-Funktionalität von 2,5 bis 3.
5) Oxadiazintriongruppen enthaltende Polyisocyanate, vorzugsweise von Hexamethylendiisocyanat oder Isophorondiisocyanat abgeleitet. Solche Oxadiazintriongruppen enthaltenden Polyisocyanate sind aus Diisocyanat und Kohlendioxid herstellbar.
6) Uretonimin-modifizierte Polyisocyanate.

Die Polyisocyanate 1) bis 6) können im Gemisch, gegebenenfalls auch im Gemisch mit Diisocyanaten, eingesetzt werden.

Als Komponente (b) kommen Verbindungen in Betracht, die mindestens eine gegenüber Isocyanat reaktive Gruppe und mindestens eine radikalisch polymerisierbare Gruppe tragen.

Gegenüber Isocyanat reaktive Gruppen können z.B. sein -OH, -SH, -NH₂ und -NHR⁹, wobei R⁹ Wasserstoff oder eine 1 bis 4 Kohlenstoffatome enthaltende Alkylgruppe, wie z.B. Methyl, Ethyl, n-Propyl, *iso*-Propyl, n-Butyl, iso-Butyl, sek-Butyl oder tert-Butyl, bedeutet.

Komponenten (b) können z.B. Monoester von α,β-ungesättigten Carbonsäuren, wie Acrylsäure, Methacrylsäure, Crotonsäure, Itaconsäure, Fumarsäure, Maleinsäure, Acrylamidoglykolsäure, Methacrylamidoglykolsäure oder Vinylether mit Di- oder Polyolen sein, die vorzugsweise 2 bis 20 C-Atome und wenigstens zwei Hydroxygruppen aufweisen, wie Ethylenglykol, Diethylenglykol, Triethylenglykol, 1,2-Propylenglykol, 1,3-Propylenglykol, 1,1-Dimethyl-1,2-Ethandiol, Dipropylenglykol, Triethylenglykol, Tetraethylenglykol, Pentaethylenglykol, Tripropylenglykol, 1,4-Butandiol, 1,5-Pentandiol, Neopentylglykol, 1,6-Hexandiol, 2-Methyl-1,5-pentandiol, 2-Ethyl-1,4-butandiol, 1,4-Dimethylolcyclohexan, 2,2-Bis(4-hydroxycyclohexyl)propan, Glycerin, Trimethylolethan, Trimethylolpropan, Trimethylolbutan, Pentaerythrit, Ditrimethylolpropan, Erythrit, Sorbit, Poly-THF mit einem Molgewicht zwischen 162 und 2000, Poly-1,3-propandiol mit einem Molgewicht zwischen 134 und 400 oder Polyethylenglykol mit einem Molgewicht zwischen 238 und 458. Weiterhin können auch Ester oder Amide der (Meth)acrylsäure mit Aminoalkoholen z. B. 2-Aminoethanol, 2-(Methylamino)ethanol, 3-Amino-1-propanol, 1-Amino-2-propanol oder 2-(2-Aminoethoxy)ethanol, 2-Mercaptoethanol oder Polyaminoalkane, wie Ethylendiamin oder Diethylentriamin, oder Vinylessigsäure verwendet werden.

Weiterhin sind auch ungesättigte Polyether- oder Polyesterole oder Polyacrylatpolyole mit einer mittleren OH-Funktionalität von 2 bis 10 geeignet.

Beispiele für Amide ethylenisch ungesättigter Carbonsäuren mit Aminoalkoholen sind
Hydroxyalkyl(meth)acrylamide wie N-Hydroxymethylacrylamid,
N-Hydroxymethylmethacrylamid, N-Hydroxyethylacrylamid,
N-Hydroyxethylmethacrylamid, 5-Hydroxy-3-oxapentyl(meth)acrylamid,
N-Hydroxyalkylcrotonamide wie N-Hydroxymethylcrotonamid oder
N-Hydroxyalkylmaleinimide wie N-Hydroxyethylmaleinimid.

Bevorzugt verwendet werden 2-Hydroxyethyl(meth)acrylat, 2- oder 3-Hydroxypropyl-(meth)acrylat, 1,4-Butandiolmono(meth)acrylat, Neopentylglykolmono(meth)acrylat, 1,5-Pentandiolmono(meth)acrylat, 1,6-Hexandiolmono(meth)acrylat, Glycerinmono- und di(meth)acrylat, Trimethylolpropanmono- und di(meth)acrylat, Pentaerythritmono-, -di- und -tri(meth)acrylat sowie 4-Hydroxybutylvinylether, 2-Aminoethyl(meth)acrylat, 2-Aminopropyl(meth)acrylat, 3-Aminopropyl(meth)acrylat, 4-Aminobutyl(meth)acrylat, 6-Aminohexyl(meth)acrylat, 2-Thioethyl(meth)acrytat, 2-Aminoethyl(meth)acrylamid, 2-Aminopropyl(meth)acrylamid, 3-Aminopropyl(meth)acrylamid, 2-Hydroxyethyl-(meth)acrylamid, 2-Hydroxypropyl(meth)acrylamid oder 3-Hydroxypropyl(meth)-acrylamid. Besonders bevorzugt sind 2-Hydroxyethylacrylat, 2-Hydroxyethylmethacrylat, 2- oder 3-Hydroxypropylacrylat, 1,4-Butandiolmonoacrylat und 3-(Acryloyloxy)-2-hydroxypropylmethacrylat.

Als Komponente (*c*) kommen Verbindungen in Betracht, die mindestens zwei gegenüber Isocyanat reaktive Gruppen, beispielsweise -OH, -SH, -NH₂ oder-NHR¹⁰, worin R¹⁰ darin unabhängig voneinander Wasserstoff, Methyl, Ethyl, *iso*-Propyl, n-Propyl, n-Butyl, *iso*-Butyl, *sek*-Butyl oder *tert*-Butyl bedeuten kann, aufweisen.

Dies sind bevorzugt Diole oder Polyole, wie 2 bis 20 Kohlenstoffatome aufweisende Kohlenwasserstoffdiole, z.B. Ethylenglycol, 1,2-Propandiol, 1,3-Propandiol, 1,1-Dimethylethan-1,2-diol, 1,6-Hexandiol, 1,10-Dekandiol, Bis-(4-hydroxycyclohexan) isopropyliden, Tetramethylcyclobutandiol, 1,2-, 1,3- oder 1,4-Cyclohexandiol, Cyclooctandiol, Norbornandiol, Pinandiol, Decalindiol, etc. deren Ester mit kurzkettigen Dicarbonsäuren, wie Adipinsäure, Cyclohexandicarbonsäure, deren Carbonate, hergestellt durch Reaktion der Diole mit Phosgen oder durch Umesterung mit Dialkyl- oder Diarylcarbonaten, oder aliphatische Diamine, wie Methylen-, und Isopropyliden-bis-(cyclohexylamin), Piperazin, 1,2-, 1,3- oder 1,4-Diaminocyclohexan, 1,2-, 1,3- oder 1,4-Cyclohexan-bis-(methylamin), etc., Dithiole oder mehrfunktionelle Alkohole, sekundäre oder primäre Aminoalkohole, wie Ethanolamin, Diethanolamin, Monopropanolamin, Dipropanolamin etc. oder Thioalkohole, wie Thioethylenglykol.

Weiterhin sind denkbar Diethylenglykol, Triethylenglykol, Dipropylenglykol, Tripropylenglykol, Neopentylglykol, Pentaerythrit, 1,2- und 1,4-Butandiol, 1,5-Pentandiol, 2-Methyl-1,5-pentandiol, 2-Ethyl-1,4-butandiol, 1,2-, 1,3- und 1,4-Dimethylolcyclohexan, 2,2-Bis(4-hydroxycyclohexyl)propan, Glycerin, Trimethylolethan, Trimethylolpropan, Trimethylolbutan, Dipentaerythrit, Ditrimethylolpropan, Erythrit und Sorbit, 2-Aminoethanol, 3-Amino-1-propanol, 1-Amino-2-propanol oder 2-(2-Aminoethoxy)ethanol, Bisphenol A, oder Butantriol.

Weiterhin sind auch ungesättigte Polyether- oder Polyesterole oder Polyacrylatpolyole mit einer mittleren OH-Funktionalität von 2 bis 10 geeignet, sowie Polyamine, wie z.B. Polyethylenimin oder freie Amingruppen enthaltende Polymere von z.B. Poly-N-vinylformamid.

Besonders geeignet sind hier die cycloaliphatischen Diole, wie z.B. Bis-(4-hydroxycyclohexan) isopropyliden, Tetramethylcyclobutandiol, 1,2-, 1,3- oder 1,4-Cyclohexandiol, Cyclooctandiol oder Norbornandiol.

Die verwendbaren Polyurethane werden durch Reaktion der Komponenten (a), (b) und (c) miteinander erhalten.

Dabei kann die molare Zusammensetzung (a):(b):(c) pro 3 mol reaktive Isocycanatgruppen in (a) in der Regel beliebig gewählt werden, bevorzugt ist sie wie folgt:
(b) 1,5 - 3,0, bevorzugt 2,0 - 2,9, besonders bevorzugt 2,0 = 2,5 und insbesondere 2,0 - 2,3 mol gegenüber Isocyanat reaktive Gruppen sowie
(c) 0 - 1,5, bevorzugt 0,1 - 1,0, besonders bevorzugt 0,5 - 1,0 und insbesondere 0,7 - 1,0 mol an gegenüber Isocyanat reaktiven Gruppen.

Bei Verwendung der Polyurethane in wäßrigen Systemen sind bevorzugt im wesentlichen alle vorhandenen Isocyanatgruppen abreagiert.

Die Bildung des Addukts aus isocyanatgruppenhaltiger Verbindung und der Verbindung, die gegenüber Isocyanatgruppen reaktive Gruppen enthält erfolgt in der Regel durch Mischen der Komponenten in beliebiger Reihenfolge, gegebenenfalls bei erhöhter Temperatur.

Bevorzugt wird dabei die Verbindung, die gegenüber Isocyanatgruppen reaktive Gruppen enthält, zu der isocyanatgruppenhaltigen Verbindung zugegeben, bevorzugt in mehreren Schritten.

Besonders bevorzugt wird die isocyanatgruppenhaltige Verbindung vorgelegt und die Verbindungen, die gegenüber Isocyanat reaktive Gruppen enthalten, zugegeben. Insbesondere wird die isocyanatgruppenhaltige Verbindung (a) vorgelegt und daraufhin (b) zugegeben. Nachfolgend können gegebenenfalls gewünschte weitere Komponenten zugegeben werden.

In der Regel wird die Reaktion bei Temperaturen zwischen 5 und 100 °C, bevorzugt zwischen 20 bis 90 °C und besonders bevorzugt zwischen 40 und 80°C und insbesondere zwischen 60 und 80 °C durchgeführt.

Bevorzugt wird dabei unter wasserfreien Bedingungen gearbeitet.

Wasserfrei bedeutet dabei, daß der Wassergehalt im Reaktionssystem nicht mehr als 5 Gew% beträgt, bevorzugt nicht mehr als 3 Gew% und besonders bevorzugt nicht mehr als 1 Gew%.

Die Reaktion kann in Gegenwart mindestens eines geeigneten Inertgases durchgeführt werden, z.B. Stickstoff, Argon, Helium, Kohlenstoffdioxid oder dergleichen, dies ist jedoch in der Regel nicht erforderlich.

Die Reaktion kann auch in Gegenwart eines inerten Solvens durchgeführt werden, z.B. Aceton, *Iso*-butyl-methylketon, Toluol, Xylol, Butylacetat oder Ethoxyethylacetat.

Die Urethan(meth)acrylate haben vorzugsweise ein zahlenmittleres Molgewicht Mₙ von 500 bis 20 000, insbesondere von 500 bis 10 000 besonders bevorzugt 600 bis 3000 g/mol (bestimmt durch Gelpermeationschromatographie mit Tetrahydrofuran und Polystyrol als Standard).

Die Urethan(meth)acrylate haben vorzugsweise einen Gehalt von 1 bis 5, besonders bevorzugt von 2 bis 4 Mol (Meth)acrylgruppen pro 1000 g Urethan(meth)acrylat.

Epoxid(meth)acrylate sind erhältlich durch Umsetzung von Epoxiden mit (Meth)acrylsäure. Als Epoxide in Betracht kommen z.B epoxidierte Olefine, aromatische Glycidylether oder aliphatische Glycidylether, bevorzugt solche von aromatischen oder aliphatischen Glycidylethern.

Epoxidierte Olefine können beispielsweise sein Ethylenoxid, Propylenoxid, iso-Butylenoxid, 1-Butenoxid, 2-Butenoxid, Vinyloxiran, Styroloxid oder Epichlorhydrin, bevorzugt sind Ethylenoxid, Propylenoxid, iso-Butylenoxid, Vinyloxiran, Styroloxid oder Epichlorhydrin, besonders bevorzugt Ethylenoxid, Propylenoxid oder Epichlorhydrin und ganz besonders bevorzugt Ethylenoxid und Epichlorhydrin.

Aromatische Glycidylether sind z.B. Bisphenol-A-diglycidylether, Bisphenol-F-diglycidylether, Bisphenol-B-diglycidylether, Bisphenol-S-diglycidylether, Hydrochinondiglycidylether, Alkylierungsprodukte von Phenol/Dicyclopentadien, z.B. 2,5-bis[(2,3-E-poxypropoxy)phenyl]octahydro-4,7-methano-5H-inden) (CAS-Nr. [13446-85-0]), Tris[4-(2,3-epoxypropoxy)phenyl]methan Isomere) CAS-Nr. [66072-39-7]), Phenol basierte Epoxy Novolake (CAS-Nr. [9003-35-4]) und Kresol basierte Epoxy Novolake (CAS-Nr. [37382-79-9]).

Aliphatische Glycidylether sind beispielsweise 1,4-Butandioldiglycidether, 1,6-Hexandioldiglycidylether, Trimethylolpropantriglycidylether, Pentaerythrittetraglycidylether, 1,1,2,2-tetrakis[4-(2,3-epoxypropoxy)phenyl]ethan (CAS-Nr. [27043-37-4]), Diglycidylether von Polypropylenglykol (α,ω-bis(2,3-epoxypropoxy)poly(oxypropylen) (CAS-Nr. [16096-30-3]) und von hydriertem Bisphenol A (2,2-bis[4-(2,3-epoxypropoxy)cyclohexyl]propan, CAS-Nr. [13410-58-7]).

Die Epoxid(meth)acrylate und -vinylether haben vorzugsweise ein zahlenmittleres Molgewicht Mₙ von 200 bis 20000, besonders bevorzugt von 200 bis 10000 g/mol und ganz besonders bevorzugt von 250 bis 3000 g/mol; der Gehalt an (Meth)acryl- oder Vinylethergruppen beträgt vorzugsweise 1 bis 5, besonders bevorzugt 2 bis 4 pro 1000 g Epoxid(meth)acrylat oder Vinyletherepoxid (bestimmt durch Gelpermeationschromatographie mit Polystyrol als Standard und Tetrahydrofuran als Elutionsmittel).

Carbonat(meth)acrylate enthalten im Mittel vorzugsweise 1 bis 5, insbesondere 2 bis 4, besonders bevorzugt 2 bis 3 (Meth)acrylgruppen und ganz besonders bevorzugt 2(Meth)acrylgruppen.

Das zahlenmittlere Molekulargewicht Mₙ der Carbonat(meth)acrylate ist vorzugsweise kleiner 3000 g/mol, besonders bevorzugt kleiner 1500 g/mol, besonders bevorzugt kleiner 800 g/mol (bestimmt durch Gelpermeationschromatgraphie mit Polystyrol als Standard, Lösemittel Tetrahydrofuran).

Die Carbonat(meth)acrylate sind in einfacher Weise erhältlich durch Umesterung von Kohlensäureestern mit mehrwertigen, vorzugsweise zweiwertigen Alkoholen (Diolen, z.B. Hexandiol) und anschließende Veresterung der freien OH-Gruppen mit (Meth)acrylsäure oder auch Umesterung mit (Meth)acrylsäureestern, wie es z.B. in EP-A 92 269 beschrieben ist. Erhältlich sind sie auch durch Umsetzung von Phosgen, Harnstoffderivaten mit mehrwertigen, z.B. zweiwertigen Alkoholen.

In analoger Weise sind auch Vinylethercarbonate erhältlich, indem man einen Hydroxyalkylvinylether mit Kohlensäureestern sowie gegebenenfalls zweiwertigen Alkoholen umsetzt.

Denkbar sind auch (Meth)acrylate oder Vinylether von Polycarbonatpolyolen, wie das Reaktionsprodukt aus einem der genannten Di- oder Polyole und einem Kohlensäureester sowie einem hydroxylgruppenhaltigen (Meth)acrylat oder Vinylether.

Geeignete Kohlensäureester sind z.B. Ethylen-, 1,2- oder 1,3-Propylencarbonat, Kohlensäuredimethyl-, -diethyl- oder -dibutylester.

Geeignete hydroxygruppenhaltige (Meth)acrylate sind beispielsweise 2-Hydroxyethyl-(meth)acrylat, 2- oder 3-Hydroxypropyl(meth)acrylat, 1,4-Butandiolmono(meth)acrylat, Neopentylglykolmono(meth)acrylat, Glycerinmono- und di(meth)acrylat, Trimethylolpropanmono- und di(meth)acrylat sowie Pentaerythritmono-, -di- und -tri(meth)acrylat.

Geeignete hydroxygruppenhaltige Vinylether sind z.B. 2-Hydroxyethylvinylether und 4-Hydroxybutylvinylether.

Besonders bevorzugte Carbonat(meth)acrylate sind solche der Formel: 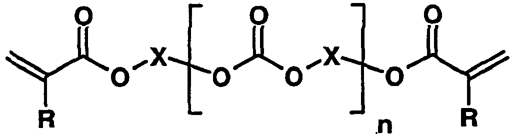 worin R für H oder CH₃, X für eine C₂-C₁₈ Alkylengruppe und n für eine ganze Zahl von 1 bis 5, vorzugsweise 1 bis 3 steht.

R steht vorzugsweise für H und X steht vorzugsweise für C₂- bis C₁₀-Alkylen, beispielsweise 1,2-Ethylen, 1,2-Propylen, 1,3-Propylen, 1,4-Butylen oder 1,6-Hexylen, besonders bevorzugt für C₄- bis C₈-Alkylen. Ganz besonders bevorzugt steht X für C₆-Alkylen.

Vorzugsweise handelt es sich bei den Carbonat(meth)acrylaten um aliphatische Carbonat(meth)acrylate.

Unter den multifunktionellen, polymerisationsfähigen Verbindung sind Urethan(meth)-acrylate besonders bevorzugt.

Ein weiterer Gegenstand der vorliegenden Erfindung sind strahlungshärtbare Beschichtungsmasse, enthaltend
- mindestens eine Verbindung der Formel (I), (II), (III), (V) oder (V1),
- mindestens eine Verbindung mit einer oder mehr als einer radikalisch polymerisierbaren Doppelbindung,
- gegebenenfalls mindestens einen Photoinitiator,
- mindestens eine Verbindungen mit mehr als einer Hydroxy- und/oder Aminogruppe,
- gegebenenfalls mindestens eine Verbindungen mit einer Hydroxy- oder Aminogruppe,
- gegebenenfalls mindestens eine organometallische Zinnverbindung oder mindestens eine Cäsiumverbindung und
- gegebenenfalls weitere lacktypische Additive.

Photoinitiatoren können beispielsweise dem Fachmann bekannte Photoinitiatoren sein, z.B. solche in "Advances in Polymer Science", Volume 14, Springer Berlin 1974 oder in K. K. Dietliker, Chemistry and Technology of UV- and EB-Formulation for Coatings, Inks and Paints, Volume 3; Photoinitiators for Free Radical and Cationic Polymerization, P. K. T. Oldring (Eds), SITA Technology Ltd, London, genannten.

In Betracht kommen z.B. Mono- oder Bisacylphosphinoxide, wie sie z.B. in EP-A 7 508, EP-A 57 474, DE-A 196 18 720, EP-A 495 751 oder EP-A 615 980 beschrieben sind, beispielsweise 2,4,6-Trimethylbenzoyldiphenylphosphinoxid (Lucirin^{®} TPO der BASF AG), Ethyl-2,4,6-trimethylbenzoylphenylphosphinat (Lucirin^{®} TPO L der BASF AG), Bis-(2,4,6-trimethylbenzoyl)-phenylphosphinoxid (Irgacure® 819 der Firma Ciba Spezialitätenchemie), Benzophenone, Hydroxyacetophenone, Phenylglyoxylsäure und ihre Derivate oder Gemische dieser Photoinitiatoren. Als Beispiele seien genannt Benzophenon, Acetophenon, Acetonaphthochinon, Methylethylketon, Valerophenon, Hexanophenon, α-Phenylbutyrophenon, p-Morpholinopropiophenon, Dibenzosuberon, 4-Morpholinobenzophenon, 4-Morpholinodeoxybenzoin, p-Diacetylbenzol, 4-Aminobenzophenon, 4'-Methoxyacetophenon, β-Methylanthrachinon, tert-Butylanthrachinon, Anthrachinoncarbonysäureester, Benzaldehyd, α-Tetraton, 9-Acetylphenanthren, 2-Acetylphenanthren, 10-Thioxanthenon, 3-Acetylphenanthren, 3-Acetylindol, 9-Fluorenon, 1-Indanon, 1,3,4-Triacetylbenzol, Thioxanthen-9-on, Xanthen-9-on, 2,4-Dimethylthioxanthon, 2,4-Diethylthioxanthon, 2,4-Di-iso-propylthioxanthon, 2,4-Dichlorthioxanthon, Benzoin, Benzoin-iso-butylether, Chloroxanthenon, Benzointetrahydropyranylether, Benzoin-methylether, Benzoin-ethylether, Benzoin-butylether, Benzoin-iso-propylether, 7-H-Benzoin-methylether, Benz[de]anthracen-7-on, 1-Naphthaldehyd, 4,4'-Bis(dimethylamino)benzophenon, 4-Phenylbenzophenon, 4-Chlorbenzophenon, Michlers Keton, 1-Acetonaphthon, 2-Acetonaphthon, 1-Benzoylcyclohexan-1-ol, 2-Hydroxy-2,2-dimethylacetophenon, 2,2-Dimethoxy-2-phenylacetophenon, 2,2-Diethoxy-2-phenylacetophenon, 1,1-Dichloracetophenon, 1-Hydroxyacetophenon, Acetophenondimethylketal, o-Methoxybenzophenon, Triphenylphosphin, Trio-Tolylphösphin, Benz[a]anthracen-7,12-dion, 2,2-Diethoxyacetophenon, Benzilketale, wie Benzildimethylketal, 2-Methyl-1-[4-(methylthio)phenyl]-2-morpholinopropan-1-on, Anthrachinone wie 2-Methylanthrachinon, 2-Ethylanthrachinon, 2-tert-Butylanthrachinon, 1-Chloranthrachinon, 2-Amylanthrachinon und 2,3-Butandion.

Geeignet sind auch nicht- oder wenig vergilbende Photoinitiatoren vom Phenylglyoxalsäureestertyp, wie in DE-A 198 26 712, DE-A 199 13 353 oder WO 98/33761 beschrieben.

Bevorzugt unter diesen Photoinitiatoren sind 2,4,6-Trimethylbenzoyldiphenylphosphinoxid, Ethyl-2,4,6-trimethylbenzoylphenylphosphinat, Bis-(2,4,6-trimethylbenzoyl)-phenylphosphinoxid, Benzophenon, 1-Benzoylcyclohexan-1-ol, 2-Hydroxy-2,2-dimethylacetophenon und 2,2-Dimethoxy-2-phenylacetophenon. Verbindungen mit mehr als einer Hydroxy- und/oder Aminogruppe sind beispielsweise die oben genannten Polyesterole, Polyetherole oder Polyacrylatpolyole.

Weiterhin kommen in Betracht Polyamine in Betracht. Hierzu geeignete Amine sind im allgemeinen polyfunktionelle Amine des Molgewichtsbereiches von 32 bis 500 g/mol, vorzugsweise von 60 bis 300 g/mol, welche mindestens zwei primäre, zwei sekundäre oder eine primäre und eine sekundäre Aminogruppe enthalten. Beispiele hierfür sind Diamine wie Diaminoethan, Diaminopropane, Diaminobutane, Diaminohexane, Piperazin, 2,5-Dimethylpiperazin, Amino-3-aminomethyl-3,5,5-trimethyl-cyclohexan (Isophorondiamin, IPDA), 4,4'-Diaminodicyclohexylmethan, 1,4-Diaminocyclohexan, Aminoethylethanolamin, Hydrazin, Hydrazinhydrat oder Triamine wie Diethylentriamin oder 1,8-Diamino-4-aminomethyloctan oder höhere Amine wie Triethylentetramin, Tetraethylenpentamin oder polymere Amine wie Polyethylen-amine, hydrierte Poly-Acrylnitrile oder zumindest teilweise hydrolysierte Poly-N-Vinylformamide jeweils mit einem Molgewicht bis zu 2000, bevorzugt bis zu 1000 g/mol.

Ferner können zum Kettenabbruch in untergeordneten Mengen Verbindungen mit einer Hydroxy- oder Aminogruppe eingesetzt werden. Sie dienen hauptsächlich zur Begrenzung des Molgewichts. Beispiele für Monoalkohole sind Methanol, Ethanol, iso-Propanol, n-Propanol, n-Butanol, iso-Butanol, sek-Butanol, tert-Butanol, Ethylenglykolmonomethylether, Ethylenglykolmonoethylether, 1,3-Propandiolmonomethylether, n-Hexanol, n-Heptanol, n-Octanol, n-Decanol, n-Dodecanol (Laurylalkohol) und 2-Ethylhexanol. Beispiele für Monoamine sind Methylamin, Ethylamin, iso-Propylamin, n-Propylamin, n-Butylamin, iso-Butylamin, sek-Butylamin, tert-Butylamin, n-Pentylamin, n-Hexylamin, n-Heptylamin, n-Octylamin, n-Decylamin, n-Dodecylamin, 2-Ethylhexylamin, Stearylamin, Cetylamin oder Laurylamin.

Als weitere lacktypische Additive können beispielsweise Antioxidantien, Stabilisatoren, Aktivatoren (Beschleuniger), Füllmittel, Pigmente, Farbstoffe, antistatische Agentien, Flammschutzmittel, Verdicker, thixotrope Agentien, oberflächenaktive Agentien, Viskositätsmodifikatoren, Plastifizierer oder Chelatbildner verwendet werden.

Weiterhin können ein oder mehrere thermisch aktivierbare Initiatoren zugesetzt werden, z.B. Kaliumperoxodisulfat, Dibenzoylperoxid, Cyclohexanonperoxid, Di-tert.-Butylperoxid, Azobis-iso-butyronitril, Cyclohexylsulfonylacetylperoxid, Di-iso-propylpercarbonat, tert-Butylperoktoat oder Benzpinakol, sowie beispielsweise solche thermisch aktivierbare Initiatoren, die eine Halbwertszeit bei 80°C von mehr als 100 Stunden aufweisen, wie Di-t-Butylperoxid, Cumolhydroperoxid, Dicumylperoxid, t-Butylperbenzoat, silylierte Pinakole, die z. B. unter dem Handelsnamen ADDID 600 der Firma Wacker kommerziell erhältlich sind oder Hydroxylgruppen-haltige Amin-N-Oxide, wie 2,2,6,6-Tetramethylpiperidin-N-oxyl, 4-Hydroxy-2,2,6,6-Tetramethylpiperidin-N-oxyl etc. Weitere Beispiele geeigneter Initiatoren sind in "Polymer Handbook", 2. Aufl., Wiley & Sons, New York beschrieben.

Als Verdicker kommen neben radikalisch (co)polymerisierten (Co)Polymerisaten, übliche organische und anorganische Verdicker wie Hydroxymethylcellulose oder Bentonit in Betracht.

Als Chelatbildner können z.B. Ethylendiaminessigsäure und deren Salze sowie β-Diketone verwendet werden.

Geeignete Füllstoffe umfassen Silikate, z. B. durch Hydrolyse von Siliciumtetrachlorid erhältliche Silikate wie Aerosil^{®} der Fa. Degussa, Kieselerde, Talkum, Aluminiumsilikate, Magnesiumsilikate, Calciumcarbonate etc.

Geeignete Stabilisatoren umfassen typische UV-Absorber wie Oxanilide, Triazine und Benzotriazol (letztere erhältlich als Tinuvin^{®} -Marken der Ciba-Spezialitätenchemie) und Benzophenone. Diese können allein oder zusammen mit geeigneten Radikalfängern, beispielsweise sterisch gehinderten Aminen wie 2,2,6,6-Tetramethylpiperidin, 2,6-Di-tert.-butylpiperidin oder deren Derivaten, z. B. Bis-(2,2,6,6-tetra-methyl-4-piperidyl)sebacinat, eingesetzt werden. Stabilisatoren werden üblicherweise in Mengen von 0,1 bis 5,0 Gew.-%, bezogen auf die in der Zubereitung enthaltenen festen Komponenten, eingesetzt.

Die Beschichtung der Substrate mit den erfindungsgemäßen Beschichtungsmassen erfolgt nach üblichen, dem Fachmann bekannten Verfahren, wobei man eine erfindungsgemäße Beschichtungsmasse oder eine solche enthaltend Lackformulierung auf das zu beschichtende Substrat in der gewünschten Stärke aufbringt und gegebenenfalls trocknet. Dieser Vorgang kann gewünschtenfalls ein- oder mehrfach wiederholt werden. Das Aufbringen auf das Substrat kann in bekannter Weise, z. B. durch Spritzen, Spachteln, Rakeln, Bürsten, Rollen, Walzen, Gießen, Laminieren, Hinterspritzen oder Coextrudieren erfolgen. Der Auftrag des Beschichtungsmittels kann auch elektrostatisch in Form von Pulver erfolgen (Pulverlacke). Die Beschichtungsstärke liegt in der Regel in einem Bereich von etwa 3 bis 1000 g/m² und vorzugsweise 10 bis 200 g/m².

Weiterhin wird ein Verfahren zum Beschichten von Substraten offenbart, bei dem man das eine erfindungsgemäßen Beschichtungsmasse oder eine solche enthaltende Lackformulierung, gegebenenfalls mit weiteren lacktypischen Additiven und thermisch, chemisch oder starhlungshärtbaren Harzen versetzt, auf das Substrat aufbringt und gegebenenfalls trocknet, mit Elektronenstrahlen oder UV Belichtung unter sauerstoffhaltiger Atmosphäre oder bevorzugt unter Inertgas härtet, gegebenenfalls bei Temperaturen bis zur Höhe der Trocknungstemperatur und anschließend bei Temperaturen bis zu 160°C, bevorzugt zwischen 60 und 160 °C, thermisch behandelt.

Das Verfahren zum Beschichten von Substraten kann auch so durchgeführt werden, daß nach dem Aufbringen der erfindungsgemäßen Beschichtungsmasse oder eine solche enthaltende Lackformulierung zunächst bei Temperaturen bis zu 160°C, bevorzugt zwischen 60 und 160 °C, thermisch behandelt und anschließend mit Elektronenstrahlen oder UV Belichtung unter Luftatmosphäre oder bevorzugt unter Inertgas gehärtet wird.

Die Härtung der auf dem Substrat gebildeten Filme kann gewünschtenfalls ausschließlich thermisch erfolgen. Im allgemeinen härtet man die Beschichtungen jedoch sowohl durch Bestrahlung mit energiereicher Strahlung als auch thermisch.

Die Härtung kann auch zusätzlich oder anstelle der thermischen Härtung durch NIR-Strahlung erfolgen, wobei als NIR-Strahlung hier elektromagnetische Strahlung im Wellenlängenbereich von 760 nm bis 2,5 µm, bevorzugt von 900 bis 1500 nm bezeichnet ist.

Gegebenenfalls kann, wenn mehrere Schichten des Beschichtungsmittels übereinander aufgetragen werden, nach jedem Beschichtungsvorgang eine NIR-, thermische und/oder Strahlungshärtung erfolgen.

Als Strahlungsquellen für die Strahlungshärtung geeignet sind z.B. Quecksilber-Niederdruckstrahler, -Mitteldruckstrahler mit Hochdruckstrahler sowie Leuchtstoffröhren, Impulsstrahler, Metallhalogenidstrahler, Elektronenblitzeinrichtungen, wodurch eine Strahlungshärtung ohne Photoinitiator möglich ist, oder Excimerstrahler. Die Strahlungshärtung erfolgt durch Einwirkung energiereicher Strahlung, also UV-Strahlung oder Tageslicht, vorzugsweise Licht im Wellenlängenbereich von λ=200 bis 700 nm strahlt, besonders bevorzugt von λ=200 bis 500 nm und ganz besonders bevorzugt λ=250 bis 400 nm, oder durch Bestrählung mit energiereichen Elektronen (Elektronenstrahlung; 150 bis 300 keV). Als Strahlungsquellen dienen beispielsweise Hochdruckquecksilberdampflampen, Laser, gepulste Lampen (Blitzlicht), Halogenlampen oder Excimerstrahler. Die üblicherweise zur Vernetzung ausreichende Strahlungsdosis bei UV-Härtung liegt im Bereich von 80 bis 3000 mJ/cm².

Selbstverständlich sind auch mehrere Strahlungsquellen für die Härtung einsetzbar, z.B. zwei bis vier. Diese können auch in jeweils unterschiedlichen Wellenlängebereichen strahlen.

Die Bestrahlung kann gegebenenfalls auch unter Ausschluß von Sauerstoff, z.B. unter Inertgas-Atmosphäre, durchgeführt werden. Als Inertgase eignen sich vorzugsweise Stickstoff, Edelgase, Kohlendioxid, oder Verbrennungsgase. Desweiteren kann die Bestrahlung erfolgen, indem die Beschichtungsmasse mit transparenten Medien abgedeckt wird. Transparente Medien sind z. B. Kunststofffolien, Glas oder Flüssigkeiten, z. B. Wasser. Besonders bevorzugt ist eine Bestrahlung in der Weise, wie sie in der DE-A 199 57 900 beschrieben ist.

Ein weiterer Gegenstand der Erfindung ist ein Verfahren zur Beschichtung von Substraten, wobei man
i) ein Substrat mit einer Beschichtungsmasse oder Lackformulierung, wie zuvor beschrieben, beschichtet,
ii) flüchtige Bestandteile der Beschichtungsmasse oder Lackformulierung zur Filmbildung unter Bedingungen entfernt, bei denen der Initiator im wesentlichen noch kleine freien Radikale ausbildet
iii) gegebenenfalls den in Schritt ii) gebildeten Film mit energiereicher Strahlung bestrahlt, wobei der Film vorgehärtet wird, und anschließend gegebenenfalls den mit dem vorgehärteten Film beschichteten Gegenstand mechanisch bearbeitet oder die Oberfläche des vorgehärteten Films mit einem anderen Substrat in Kontakt bringt,
iv) den Film thermisch endhärtet.

Dabei können die Schritte iv) und iii) auch in umgekehrter Reihenfolge durchgeführt, d. h. der Film kann zuerst thermisch und dann mit energiereicher Strahlung gehärtet werden.

In dieser Schrift verwendete ppm- und Prozentangaben beziehen sich, falls nicht anders angegeben, auf Gewichtsprozente und -ppm.

Die folgenden Beispiele sollen die Erfindung erläutern, aber nicht auf diese Beispiele einschränken.

### Beispiele

### Vergleichsbeispiel 1

In einem 250-ml-Vierhals-Reaktionskolben, ausgestattet mit Destillierbrücke und Liebigkühler und Rührer, wurden in 4,74 g n-Butylacetat 6,0 g 2,4,6-Tris(methylcarbamoyl)-1,3,5-triazin, 6,97 g 2-Hydroxyethylacrylat, 12,5 mg 4-Methoxyphenol, 4 mg 2,6-Di-t-butyl-p-kresol und 0,3 mg Phenothiazin gelöst und auf eine Innentemperatur von 110 °C gebracht. Das entstehende Methanol wurde abdestilliert.

Umsatz nach 300 Minuten: 25 % des 2-Hydroxyethylacrylats, 4 % 2,4,6-Tris(2-ethoxyacrylato-carbamoyl)-1,3,5-triazin (HPLC).

### Beispiel 1

In einem 250-ml-Vierhals-Reaktionskolben, ausgestattet mit Destillierbrücke und Liebigkühler und Rührer, wurden in 12,32 g n-Butylacetat 6,0 g 2,4,6-Tris(methylcarbamoyl)-1,3,5-triazin, 6,97 g 2-Hydroxyethylacrylat, 12,5 mg 4-Methoxyphenol, 4 mg 2,6-Di-t-butyl-p-kresol und 0,3 mg Phenothiazin sowie 0,96 mg Cäsiumacetat gelöst und auf eine Innentemperatur von 110 °C gebracht. Das entstehende Methanol wurde abdestilliert.

Umsatz nach 300 Minuten: 50 % des 2-Hydroxyethylacrylats, 17 % 2,4,6-Tris(2-ethoxyacrylato-carbamoyl)-1,3,5-triazin (HPLC).

### Beispiel 2

Eine Suspension aus 0,136 g p-Methoxyphenol, 0,045 g di-tert-Butyl-p-kresol, 0,003 g Phenothiazin, 0,016 g Dibutylzinndilaurat (DBTL), 2,4,6-Trisalkoxycarbamoyl-1,3,5-triazin, Diol und/oder Polyesterol sowie Hydroxyethylacrylat (HEA) wie in der Tabelle angegeben in 30,0 ml Methylisobutylketon (MIBK) wurde 4 Stunden bei 112 °C Badtemperatur gerührt. Anschließend wurde die Reaktionsmischung bei 52 °C Badtemperatur und einem Vakuum von 750 mbar 2 Stunden destilliert. Es wurde eine klare Harzlösung erhalten. Die eingesetzten molaren Mengen sind Tabelle 2 zu entnehmen.

### Filmprüfungen

Die Harzlösungen wurden durch Zugabe von Methylisobutylketon auf eine Viskosität von ca. 3,5 Pas eingestellt und mit 4 Gewichtsprozent (bezogen auf den Feststoffgehalt) 2-Hydroxy-2-methyl-1-phenyl-propan-1-on als Photoinitiator (Darocur^{®} 1173 der Firma Ciba Spezialitätenchemie) gemischt. Die Beschichtungsmassen wurden mit Hilfe einer Kastenrakel auf das jeweilige Substrat aufgetragen und zum Entfernen des Lösemittels 30 Minuten bei 60 °C getrocknet.

Die Beschichtungen wurden durch 30 minütiges Tempern thermisch gehärtet oder unter einer undotierten Quecksilberhochdrucklampe (Leistung 120 W/cm) mit einem Lampenabstand zum Substrat von 12 cm und einer Bandgeschwindigkeit von 10 m/min etwa bei einer Temperatur von 100 °C belichtet oder zuerst belichtet und anschließend thermisch gehärtet.

Die Pendelhärte (PD) wurde nach DIN 53157 bestimmt und ist ein Maß für die Härte der Beschichtung. Die Angabe erfolgt in Sekunden bis zum Stillstand des Pendels (s). Hohe Werte bedeuten dabei hohe Härte. Die Filme zur Bestimmung der Pendelhärte wurden mit Hilfe einer Kastenrakel auf Glas aufgetragen. Die Schichtdicke vor der Härtung betrug 100 µm.

Die Erichsentiefung (ET) wurde nach DIN 53156 bestimmt und ist ein Maß für die Flexibilität und Elastizität. Die Angabe erfolgt in Millimeter (mm). Hohe Werte bedeuten hohe Flexibilität. Die Filme zur Bestimmung der Erichsentiefung wurden mit Hilfe einer Spiralrakel auf Blech aufgetragen. Die Schichtdicke vor der Härtung beträgt 50 µm.

**Tabelle 1**

| Beispiel | 2,4,6-Tris(alkoxy-carbamoyl)-1,3,5-triazin (mol) | HEA (mol) | Diol (mol) | Härtung | PD(s) | ET (mm) |
|---|---|---|---|---|---|---|
| | | | | therm. | 8 | 9,8 |
| 2 | 0,4 ¹⁾ | 1,2 | 0,2 A | photochem. | 10 | 9,9 |
| | | | | photochem.+therm. | 39 | 9,8 |
| | | | | therm. | 17 | 9,8 |
| 3 | 0,2¹⁾ | 0,6 | 0,05 A | photochem. | 21 | 9,8 |
| | | | | photochem.+therm. | 76 | 9,4 |
| | | | | therm. | 54 | 9,7 |
| 4 | 0,1¹⁾ | 0,6 | 0,025 A | photochem. | 36 | 7,2 |
| | | | | photochem.+therm. | 170 | 7,3 |
| | | | | therm. | 46 | 5,1 |
| 5 | 0,1²⁾ | 0,3 | - | photochem. | 200 | 1,2 |
| | | | | photochem.+therm. | 235 | 2,1 |
| | | | | therm. | 60 | 9,8 |
| 6 | 0.1 ²⁾ | 0,3 | 0,125 A | photochem. | 83 | 6,1 |
| | | | | photochem.+therm. | 182 | 1,1 |
| | | | | therm. | 126 | 5,1 |
| 7 | 0,4 ²⁾ | 1,2 | 0,008 A | photochem. | 153 | 1,1 |
| | | | | photochem.+therm. | 235 | 3,8 |
| | | | | therm. | 221 | 3,5 |
| 8 | 0,4 ²⁾ | 1,2 | 0,2 B | photochem. | 161 | 2,3 |
| | | | | photochem.+therm. | 242 | 2,1 |
| | | | 6,7 A | therm. | 66 | 4,5 |
| 9 | 0,1 ²⁾ | 0,3 | 0,0067 B | photochem. | 129 | 1,1 |
| | | | | photochem.+therm. | 210 | 1,1 |

| | | | | | | |
|---|---|---|---|---|---|---|
| 1) 2,4,6-Tris(methoxycarbamoyl)-1,3,5-triazin 2) 2,4,6-Tris(methoxy/butoxy-carbamoyl)-1,3,5-triazin (molares Verhältnis Methyl:Butyl=60:40) A Polyester aus 1 mol Adipinsäure, 1 mol Isophthalsäure und 2 mol 1,6-Hexandiol, Molmasse ca. 1000 g/mol B 1,4-Butandiol | | | | | | |

Die Beispiele zeigen, dass eine vollständige Härtung erst durch Kombination von thermischer und photochemischer Vernetzung erreicht wird. Auf diese Weise erhält man mit den erfindungsgemäßen Verbindungen außergewöhnlich harte Beschichtungen.

## Patentansprüche

1. 1,3,5-Triazincarbamate und -harnstoffe der Formel (I), 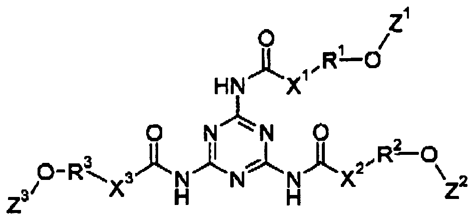 worin
R¹, R² und R³ jeweils unabhängig voneinander einen zweiwertigen organischen Rest,
X¹, X² und X³ jeweils unabhängig voneinander Sauerstoff oder substituierter oder unsubstituierter Stickstoff (NR),
R Wasserstoff oder C₁ - C₂₀-Alkyl und
Z¹, Z² und Z³ jeweils unabhängig voneinander Vinyl, Methacryloyl oder Acryloyl bedeuten.

2. 1,3,5-Triazincarbamate und -harnstoffe der Formel (II) 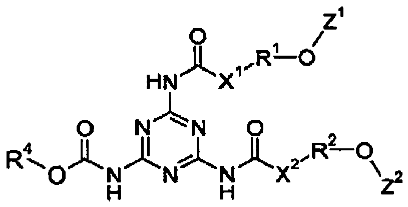 und der Formel (III) 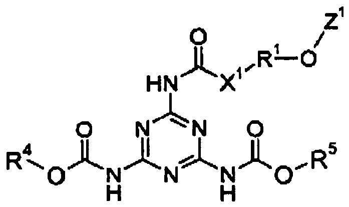 worin
X¹, X², Z¹, Z², R¹ und R² die gleiche Bedeutung haben, wie in Anspruch 1 genannt, und
R⁴ und R⁵ jeweils unabhängig voneinander C₁ - C₄-Alkyl
bedeuten.

3. Isocyanatgruppenhaltige 1,3,5-Triazincarbamate und -harnstoffe der Formel (V) 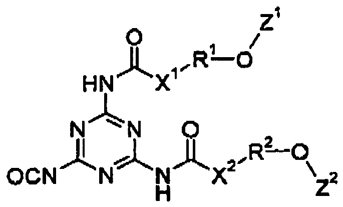 und Formel (VI) 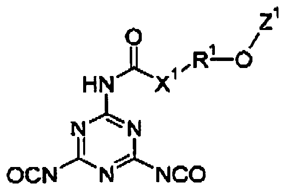 worin
X¹, X², Z¹, Z², R¹ und R² die gleiche Bedeutung haben, wie in Anspruch 1 genannt.

4. Verfahren zur Herstellung von Verbindungen der Formel (I), (II) und (III) gemäß Anspruch 1 oder 2
durch Umsetzung von Verbindungen der Formel (IV), 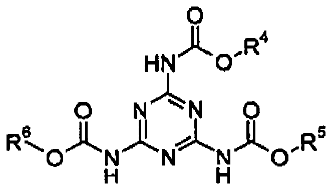 worin
R⁴, R⁵ und R⁶ jeweils unabhängig voneinander C₁ - C₄-Alkyl sein kann,
mit Alkoholen oder Aminen der Formel (VII)
Z¹-O-R¹-X¹-H, bzw. Z²-O-R²-X²-H oder Z³-O-R³-X³-H,
worin X¹, X², X³, Z¹, Z², Z³, R¹, R² und R³ die gleiche Bedeutung haben, wie in Anspruch 1 genannt.

5. Verfahren zur Herstellung von Verbindungen der Formel (I), (II) und (III) wie in Anspruch 1 oder 2 definiert durch Umsetzung von 2,4,6-Triisocyanato-[1,3,5]triazin mit Alkoholen oder Aminen der Formel (VII), wie in Anspruch 4 definiert, und im Falle der Verbindungen (II) und (III) gleichzeitiger, vorheriger oder anschließender Umsetzung mit Alkoholen der Formel R⁴OH oder R⁵OH, worin R⁴ und R⁵ jeweils unabhängig voneinander C₁ -C₄-Alkyl sein kann.

6. Verfahren zur Herstellung von Verbindungen der Formeln (V) oder (VI) gemäß Anspruch 3 durch Umsetzung von 2,4,6-Triisocyanato-[1,3,5]triazin mit Alkoholen oder Aminen der Formel (VII) wie in Anspruch 4 definiert.

7. Beschichtungsmittel, enthaltend mindestens eine Verbindung der Formel (I) und/oder Formel (II) und/oder Formel (III) und/oder Formel (V) und/oder Formel (VI) wie in Anspruch 1, 2 oder 3 definiert.

8. Verwendung von Verbindungen der Formel (I) wie in Anspruch 1 definiert in der Strahlungshärtung.

9. Verwendung von Verbindungen der Formel (II) und/oder Formel (III) und/oder Formel (V) und/oder Formel (VI) wie in Anspruch 2 oder 3 definert in der Dual-Cure-Härtung.

## Claims

1. A 1,3,5-triazine carbamate or 1,3,5-triazine urea of formula (I) 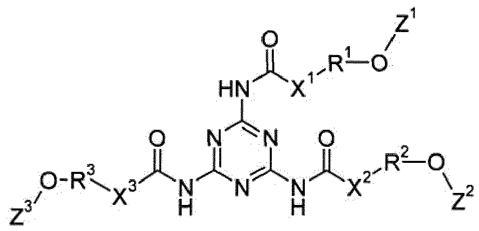 in which
R² and each independently of one another are a divalent organic radical,
X¹, X² and X³ each independently of one another are oxygen or substituted or unsubstituted nitrogen (NR),
R being hydrogen or C₁ - C₂₀alkyl, and
Z¹, and Z³ each independently of one another are vinyl, methacryloyl or acryloyl.

2. A 1,3,5-triazine carbamate or 1,3,5-triazine urea of formula (II) 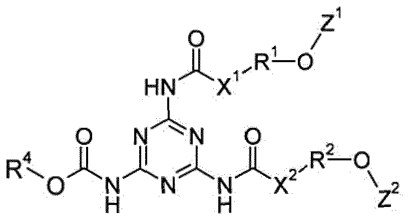 or of formula (III) 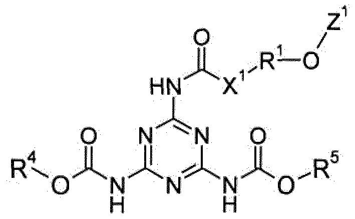 in which
X¹, X², Z¹, Z², R¹ and R² are as defined in claim 1 and
R⁴ and R⁵ each independently of one another are C₁ - C₄ alkyl.

3. An isocyanato-functional 1,3,5-triazine carbamate or 1,3,5-triazine urea of formula (V) 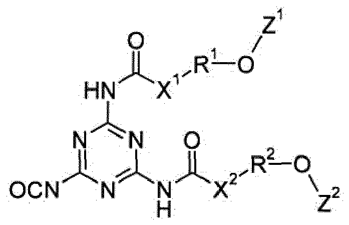 or formula (VI) 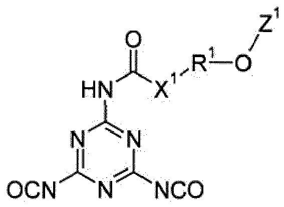 in which
X¹, Z¹, Z², R¹ and R² are as defined in claim 1.

4. A process for preparing a compound of formula (I), (II) or (III) as set forth in claim 1 or 2
by reacting a compound of formula (IV) 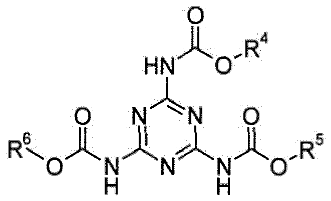 in which
R⁴, R⁵ and R⁶ in each case independently of one another can be C₁ - C₄ alkyl
with an alcohol or amine of formula (VII)
Z¹-O-R1-X¹-H or Z²-O-R²-X²-H, or 2³-O-R³-X³-H,
in which X¹, X², X³, Z¹, Z², Z³, R¹, R² and R³ are as defined in claim 1.

5. A process for preparing a compound of formula (I), (II) or (III) as defined in claim 1 or 2 by reacting 2,4,6-triisocyanato-1,3,5-triazine with an alcohol or amine of formula (VII), as defined in claim 4, and in the case of compound (II) or (III) by simultaneous, prior or subsequent reaction with alcohols of formula R⁴OH or R⁵OH, where and R⁵ each independently of one another can be C₁ - C₄ alkyl.

6. A process for preparing a compound of formula (V) or (VI) as set forth in claim 3 by reacting 2,4,6-triisocyanato-1,3,5-triazine with an alcohol or amine of formula (VII) as defined in claim 4.

7. A coating composition comprising at least one compound of formula (I) and/or formula (II) and/or formula (III) and/or formula (V) and/or formula (VI) as defined in claim 1, 2 or 3.

8. The use of a compound of formula (I) as defined in claim 1 in radiation curing.

9. The use of a compound of formula (II) and/or formula (III) and/or formula (V) and/or formula (VI) as defined in claim 2 or 3 in dual-cure curing.

## Revendications

1. 1,3,5-triazinecarbamates et -urées de formule (I), 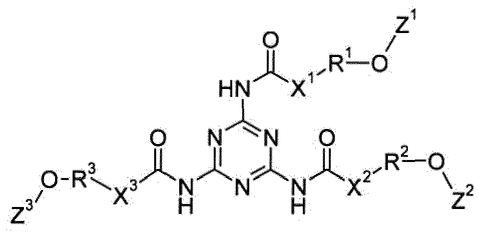 dans laquelle
R¹, R² et R³ représentent chacun indépendamment un radical organique divalent,
X¹, X² et X³ représentent chacun indépendamment un atome d'oxygène ou un atome d'azote non substitué ou substitué (NR),
R représente un atome d'hydrogène ou un groupe alkyle en C₁-C₂₀ et
Z¹, Z² et Z³ représentent chacun un groupe vinyle, méthacryloyle ou acryloyle.

2. 1,3,5-triazinecarbamates et -urées de formule (II), 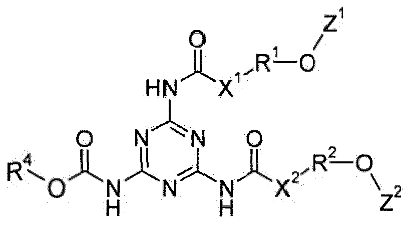 ou de formule (III) 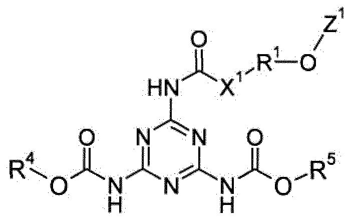 dans lesquelles
X¹, X², Z¹ , Z², R¹ et R² ont la même signification que celle indiquée dans la revendication 1, et
R⁴ et R⁵ représentent, indépendamment l'un de l'autre, un groupe alkyle en C₁-C₄.

3. 1,3,5-triazinecarbamates est -urées contenant des groupes isocyanate, de formule (V), 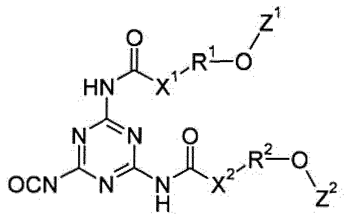 et de formule (VI) 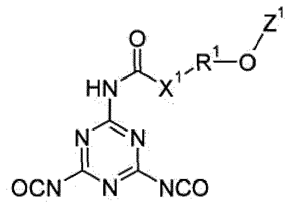 dans lesquelles
X¹, X², Z¹, Z², R¹ et R² ont la même signification que celle indiquée dans la revendication 1.

4. Procédé pour la préparation de composés de formules (I), (II) et (III) selon la revendication 1 ou 2,
par mise en réaction de composés de formule (IV), 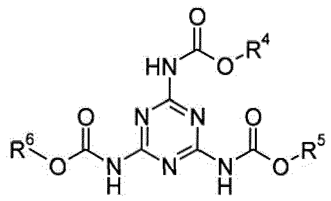 dans laquelle
R⁴, R⁵ et R⁶ peuvent être chacun indépendamment un groupe alkyle en C₁-C₄,
avec des alcools ou des amines de formule (VII)
Z¹-O-R¹-X¹-H ou Z²-O-R²-X²-H ou Z³-O-R³-X³-H,
où X¹, X², X³, Z¹, Z², Z³, R¹, R² et R³ ont la même signification que celle indiquée dans la revendication 1.

5. Procédé pour la préparation de composés de formules (I), (II) p et (III) telles que définies dans la revendication 1 ou 2, par mise en réaction de 2,4,6-triisocyanato-[1,3,5]triazine avec des alcools ou des amines de formule (VII), telle que définie dans la revendication 4, et dans le cas des composés (II) et (III), réaction simultanée, préliminaire ou subséquente avec des alcools de formule R⁴OH ou R⁵OH, où R⁴ et R⁵ peuvent être chacun, indépendamment l'un de l'autre, un groupe alkyle en C₁-C₄.

6. Procédé pour la préparation de composés de formules (V) ou (VI) selon la revendication 3, par mise en réaction de 2,4,6-triisocyanato-[1,3,5]triazine avec des alcools ou des aminés de formule (VII), telle que définie dans la revendication 4.

7. Composition de revêtement, contentant au moins un composé de formule (I) et/ou de formule (II) et/ou de formule (III) et/ou de formule (V) et/ou de formule (VI), telles que définies dans la revendication 1, 2 ou 3.

8. Utilisation des composés de formule (I) telle que définie dans la revendication 1, dans le durcissement par irradiation.

9. Utilisation des composés de formule (II) et/ou de formule (III) et/ou de formule (V) et/ou de formule (VI), telles que définies dans la revendication 2 ou 3, dans le durcissement Dual-Cure.
